(19) Europäisches Patentamt
European Patent Office
Office européen des brevets

(11) Publication number: **0 408 790 A1**

(12) **EUROPEAN PATENT APPLICATION**

(21) Application number: **89113310.0**

(51) Int. Cl.5: **C12N 15/12**, C12P 21/02

(22) Date of filing: **20.07.89**

Claims 11 - 19 are deemed to be abandoned due to non-payment of the claims fee (Rule 31 (2) EPC).

(43) Date of publication of application:
**23.01.91 Bulletin 91/04**

(84) Designated Contracting States:
**GB**

(71) Applicant: **Taniguchi, Tadatsugu, Dr.**
**Institute for Molecular and Cellular Biology**
**Osaka University 1-3, Yamadaoka**
**Suita-shi Osaka 565(JP)**

Applicant: **Miyasaka, Masayuki, Dr.**
**Department of Immunology The Tokyo**
**Metropolitan Institute of Medical Science**
**Bunkyo-ku Tokyo 113(JP)**

Applicant: **Tsudo, Mitsuru**
**Department of Immunology The Tokyo**
**Metropolitan Institute of Medical Science**
**Bunkyo-ku Tokyo 113(JP)**

(72) Inventor: **Tsudo, Mitsuru, Dr.**
**3-37-1-204, Narimasu**
**Itabashi-ku Tokyo 175(JP)**
Inventor: **Karasuyma, Hajime, Dr.**
**5-21-38-116, minami-Ooizumi**
**Nerima-ku Tokyo 178(JP)**

(74) Representative: **Laudien, Dieter, Dr. et al**
**Boehringer Ingelheim Zentrale GmbH ZA**
**Patente Postfach 200**
**D-6507 Ingelheim am Rhein(DE)**

(54) **Recombinant protein receptor.**

(57) Recombinant IL-21R$\beta$ chain or portions thereof, cDNA coding therefore, vectors containing said cDNA, hosts transfected by said vectors, and monoclonal antibodies to said recombinant IL-2R$\beta$ or portions thereof.

EP 0 408 790 A1

# RECOMBINANT PROTEIN RECEPTOR

This invention relates to receptors for interleukin-2, more particularly to the β-chain of the receptor and parts there- of, and to cDNA coding for the β-chain or parts thereof, vectors containing cDNA inserts coding for the β-chain and parts thereof, hosts transformed by such vectors and the cultivation of such hosts to produce the said β-chain and parts thereof.

Ample evidence has been accumulated that cytokines, a class of soluble mediators involved in cell-to-cell "communications", are essential in the regulation of the immune system. It has been known that cytokines induce proliferation, differentiation and activation of target cells through interaction with specific cell surface receptor(s). Interleukin-2 (IL-2), previously defined as T cell growth factor (1), is one of the best characterized cytokines, known to play a pivotal role in the antigen-specific clonal proliferation of T lymphocytes (T cells) (2). IL-2 also appears to act on other cells of the immune system such as immature thymocytes (3), B lymphocytes (B cells) (4), macrophages (5), natural killer cells (NK cells) (6), and lymphokine-activated killer cells (LAK cells) (7). These multifunctional properties of IL-2 have now opened up possibilities in the formulation of immunotherapies such as adoptive immunotherapy (8). More recently, IL-2 has been shown to function also on neural cells such as oligodendrocytes (9), suggesting a possible involve ment of this cytokine in the central nervous system. Despite extensive studies on the IL-2 system in the context of basic and clinical immunology, information has been limited on the molecular mechanism(s) underlying the IL-2-mediated signal transduction (10).

The IL-2 receptor (IL 2R) is known to be unique in that it is present in three forms: high-, intermediate- and low-affinity forms with respect to its binding ability to IL-2, and respective dissociation constants (Kds) of $10^{-11}$M, $10^{-9}$M and $10^{-8}$M (11, 12). Following the characterization of IL-2Rα chain (Tac antigen, p55) (13), it became evident that the α chain constitutes solely the low-affinity form and it is not functional per se in IL-2 internalization and signal transduction, unless associated with another specific membrane component(s) of lymphoid cells (14, 15). Subsequently, the lymphoid membrane component was identified to be a novel receptor chain, termed β chain (or p70-75) (12, 16, 17). In fact, experimental evidence has suggested that the IL-2Rβ chain per se constitutes the intermediate-affinity form (12). In addition, its association with the IL-2Rα chain results in the high-affinity form of the receptor (12, 16, 17). Expression studies using wild type and mutated IL-2Rα chain cDNAs strongly support the notion that the IL-2Rβ chain but not the IL-2Rα chain possesses a domain(s) responsible in driving the intracellular signal transduction pathway(s) (18). There exists therefore a need to obtain IL-2β chain in amounts which will enable its structure and function to be elucidated, this being an essential step in gaining further insight into the molecular basis of the high-affinity IL-2R as well as on the mechanism of signal transduction operating in IL-2 responsive cells. To this end we describe below cDNA coding for the IL-2Rβ chain or parts thereof whereby insertion of said cDNA in a suitable vector and expression thereof in an appropriate host will enable recombinant and large scale production of protein corresponding to the IL-2Rβ chain or parts thereof.

Isolation and analysis of the cDNA clones coding for human IL-2Rβ chain

In isolating the cDNA clones, we applied an expression cloning strategy by using the monoclonal antibodies, Mik-β1 and Mik-β2 (19), both of which have been raised against the IL-2Rβ chain found on the human leukemic cell line (YT) (20). The monoclonal antibodies Mik-β1 and Mik-β2 are both deposited at Fermentation Research Institute, Agency of Industrial Science and Technology, Japan. The deposit numbers for Mik-β1 and Mik-β2 are, 10453 and 10454 (1988), respectively; they are also described in Japanese Patent Application No. 298742 (1988).

A few sets of cDNA libraries were prepared by using the poly(A)$^{+}$-RNA from YT cells according to standard procedures. cDNA libraries were prepared with cDM8 vector according to published procedures (21), except using random primer (Amersham) or oligo (dT) primer as mentioned below. The plasmid DNA representing $5.6 \times 10^{6}$ independent colonies were prepared by the standard procedure and one mg of DNA were used for the first DNA transfection. Actually, the DNA was divided into 100 tubes (therefore each tube contained 10 μg of DNA) and they were each transfected into $3.5 \times 10^{5}$ monkey COS cells in a tissue culture dish (60 mm polystyrene dish, Corning). The transfection was done using the standard DEAE dextran procedures. The transfected COS cells were then treated with the cocktail of Mik-β1 and -β2 antibodies (400-fold diluted ascites for each antibody) and subjected to the standard panning procedure. The dish used for the panning was FALCON 60 mm dish, coated with anti-mouse IgG as described previously (ref.

21). In this first round of panning, 100 IgG-coated dishes were used. After the panning, Hirt extract was prepared by the standard procedure (ref. 21) and the recovered plasmids were introduced into E.coli by the method described in ref. 21. By this procedure $3.7 \times 10^6$ colonies were obtained. Those bacterial colonies were fused with COS cells by the standard protoplast fusion procedures (ref. 21). In these fusion experiments, 26 Corning dishes each containing $5 \times 10^5$ COS cells were used. After the fusion, the COS cells were subjected to panning as described above and Hirt extract was prepared. 32,000 bacterial colonies were obtained from the Hirt extract. The fusion, panning procedures were repeated again and 32,000 bacterial colonies were obtained from the subsequent Hirt extract. The same procedure were repeated once again, obtaining 28,000 bacterial colonies (in the meantime, there should be a dramatic enrichment of the objective clones). The same procedures were repeated once again and 6,000 colonies were obtained. From these colonies, 30 colonies were picked up randomly and the cDNA inserts were analysed. Of them, only 7 colonies contained plasmids from which cDNA inserts can be excised by restriction enzyme XhoI. The vector derived XhoI sites are located at both sides of the cDNA and all other plasmids had lost such cleavage sites due to the DNA rearrangements; in fact, all of them were much smaller in size than the original vector. Thus they were considered to be non-specific products. On the other hand, all of the 7 colonies were derived from the same mRNA, as confirmed by the conventional restriction enzyme cleavage analysis and DNA blot analysis. Of them, one plasmid, termed pIL-2Rβ30 contained longer cDNA than the other 6 plasmids which turned out to be identical to each other (designated gTT-2Rβ9).

In this procedure, therefore, we isolated two independent cDNA clones, pIL-2Rβ9 and pIL-2β30; each of the expression products specifically reacted with the antibodies. The two clones contained cDNA inserts of 1.3Kb and 2.3Kb, respectively, and cross-hybridized with each other. Subsequent sequence analysis of the cDNAs revealed that they represent the same mRNA. In fact, RNA blotting analysis revealed that the mRNA is approximately 4Kb in size (see below). Subsequently, we screened other YT cDNA libraries by using the cloned cDNAs as probes, and several independent cDNA clones which together cover the entire mRNA for the IL-2Rβ chain were isolated. Thus pIL-2Rβ6 and pIL-2Rβ19 were obtained by screening the cDNA libraries with the pIL-2Rβ9 cDNA insert in the probe.

The above mentioned plasmids containing cDNA coding for human IL-2Rβ sequences have been deposited in strain E.coli MC 1061/P3 on March 2, 1989 at the Fermentation Research Institute according to the Budapest Treaty under the following accession numbers:

| Plasmid | Accession No. |
|---|---|
| pIL-2Rß6 | FERM BP-2312 |
| pIL-2Rß9 | FERM BP-2313 |
| pIL-2Rß19 | FERM BP-2314 |
| pIL-2Rß30 | FERM BP-2315 |

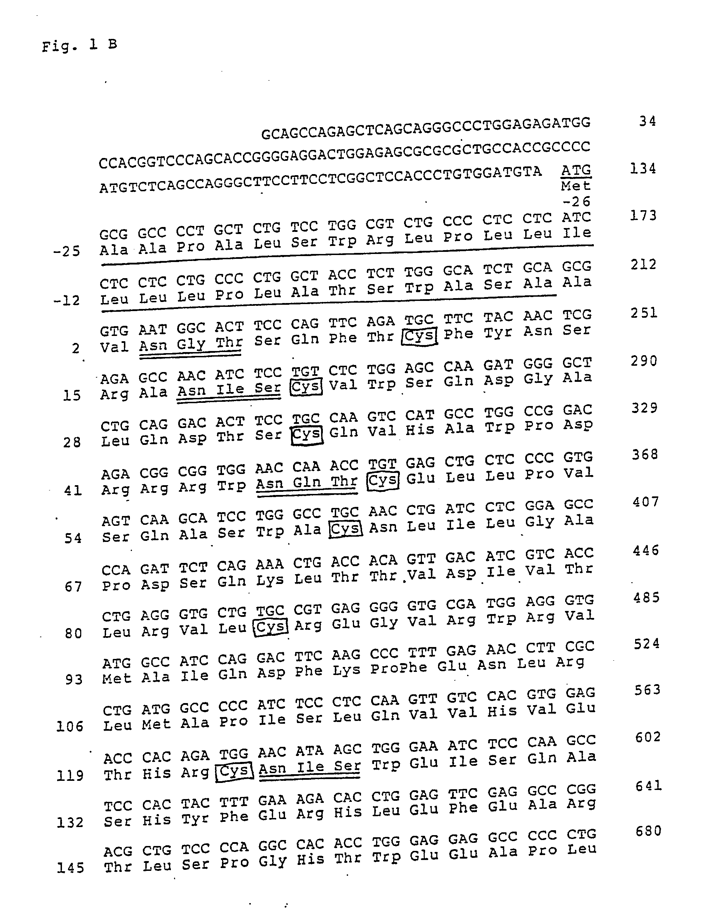

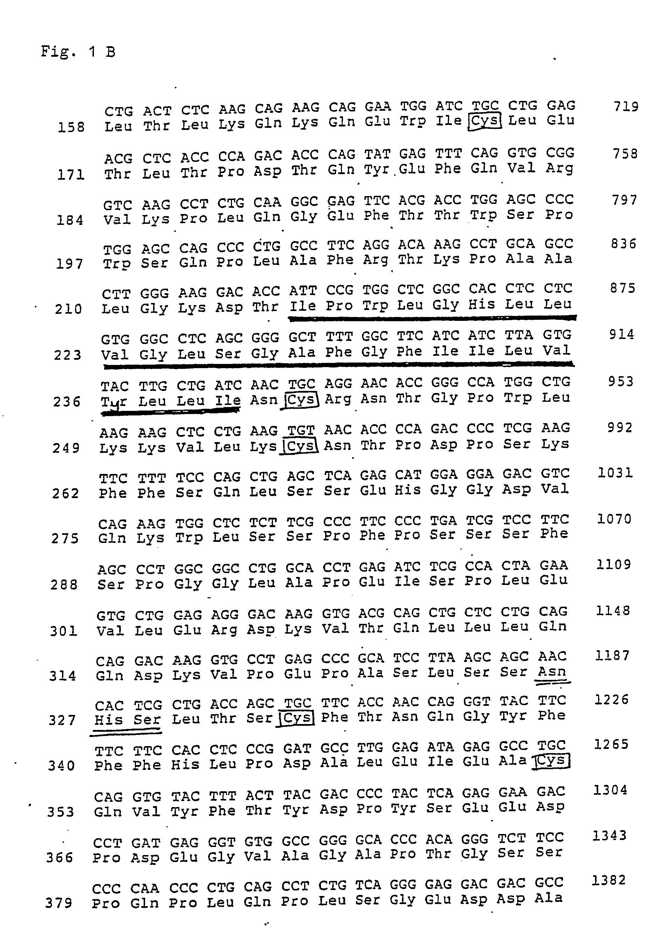

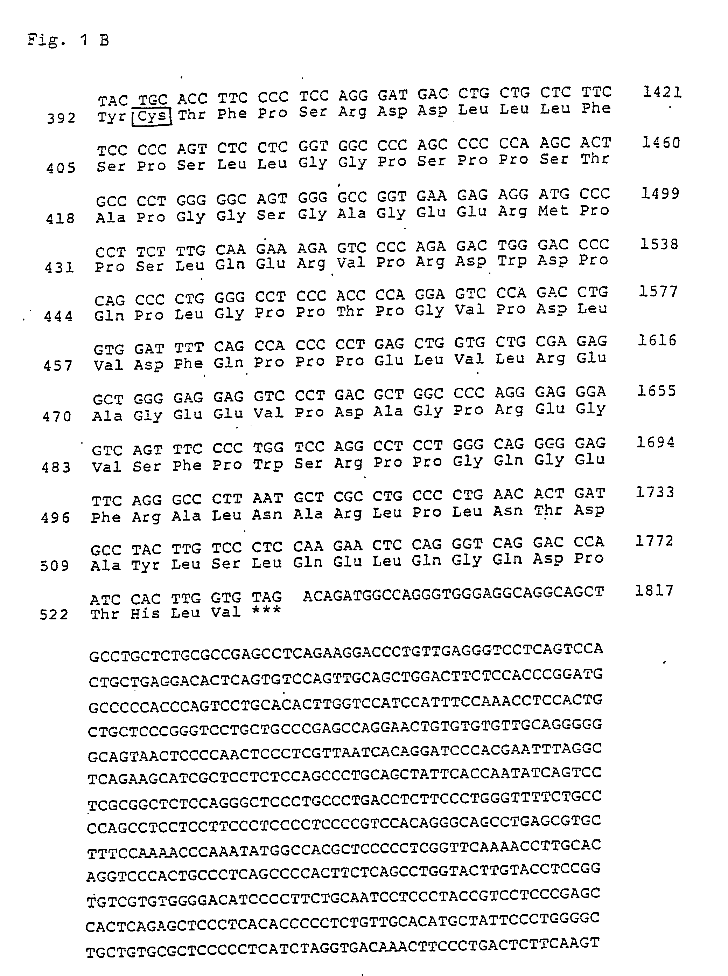

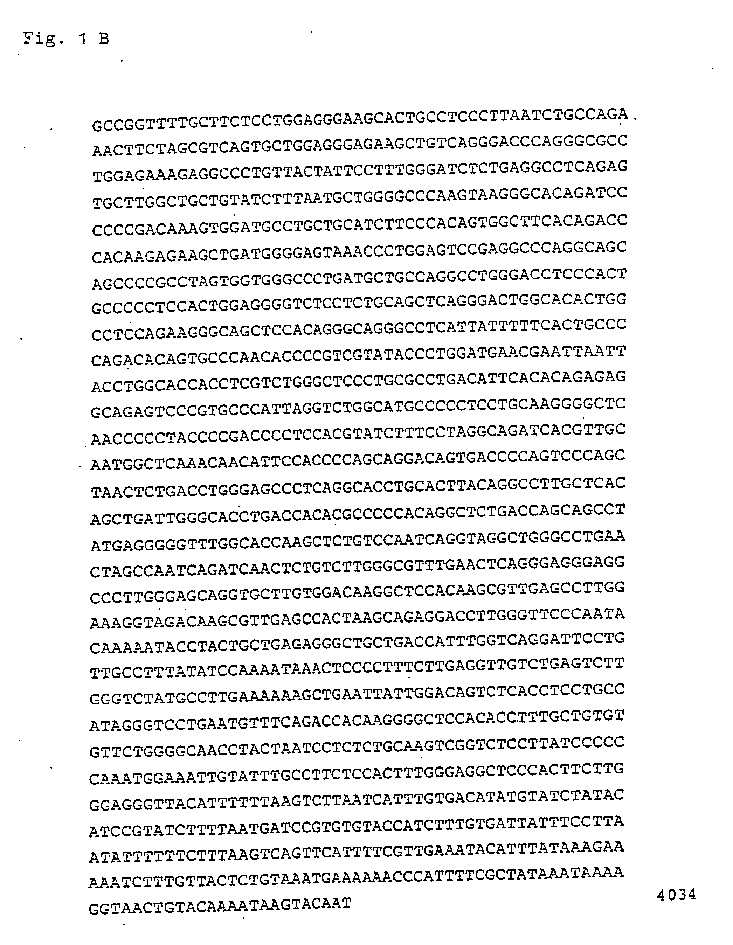

The complete nucleotide sequences of four of the cloned cDNAs were determined (Fig. 1).

Fig. 1 shows the structure of the human IL-2Rβ chain cDNA. Fig. 1a is a schematic representation of the mRNA as deduced from the cloned cDNAs. Dotted, hatched, open and closed rectangles represent respectively the signal sequence, the ex tracellular, the transmembrane and the cytoplasmic regions of the mRNA are shown below. Fig. 1b shows the nucleotide and amino acid sequences of the human IL-2Rβ chain cDNA. The sequence was deduced following the complete DNA sequence analysis of the above described cDNA clones. Nucleotides are numbered on the right margin and amino acids are numbered on the left margin. Clones pIL-2Rβ19 and pIL-2Rβ6 each contained G-A mutation at nucleotide residues 425 and 1531, respectively. Thus pIL-2Rβ6 cDNA acquired a TAG triplet in the cytoplasmic region. It is thought to be an error in reverse transcription, since all other clones, pIL-2Rβ30, pIL-2Rβ19 and pIL-2Rβ16 (28), have TGG triplet at that position. The first underlined 26 amino acid residues represent the signal sequence as predicted by the consensus sequence (22). The 25 transmembrane amino acid residues are marked with a thick underlining. The cysteine residues are boxed. The potential N-glycosylation sites are underlined twice. The possible poly-adenylation signals are shown by open rectangle. Nucleotide sequences were determined by a combination of dideoxy chain termination and chemical cleavage methods.

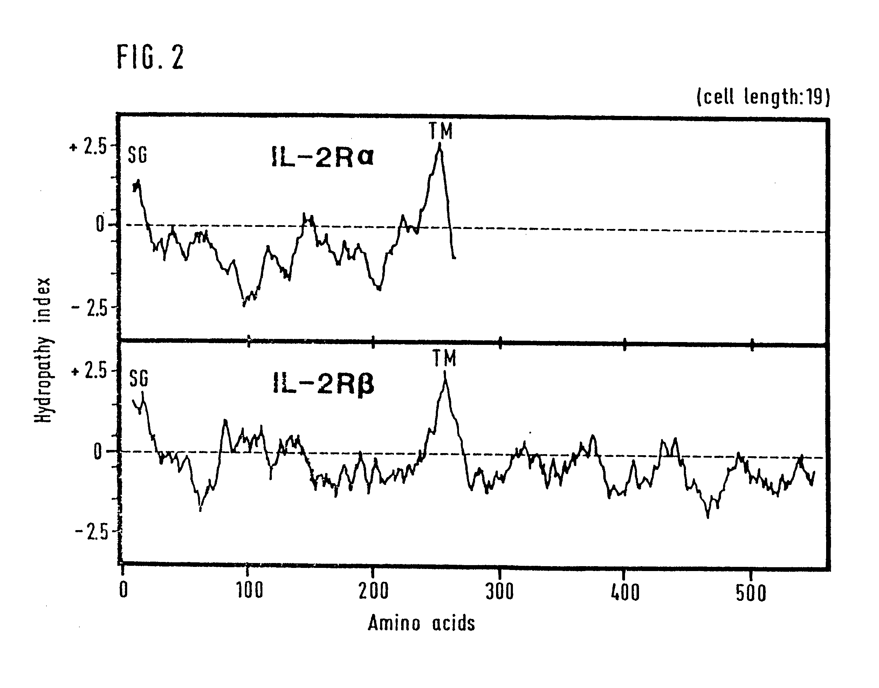

As shown in Fig. 1, the cDNA contains a large open reading frame that encodes a protein consisting of 551 amino acids. No significant homology with other known proteins was found in the Protein Sequence Database (National Biomedical Research Foundation, Washington, D.C.) or with sequences published more recently. Unlike many of the other cytokine receptors, it appears that IL-2Rα and Il-2Rβ chains do not belong to the immunoglobulin superfamily. From the deduced structure of the protein, the N-terminal 26 amino acids is considered to represent the signal sequence (Fig. 1 and 2) (22). Thus the mature form of the IL-2Rβ chain is considered to consist of 525 amino acids with a calculated M.W. of 58.358. As shown in Fig. 1, the receptor molecule consists of an extracellular region consisting of 214 amino acids. This region contains 8 cysteine residues of which 5 residues are found in the N-terminal half and they are interspaced rather periodically by 9-12 amino acids. It is likely that disulfide linkages between the cysteine residues impart a stable configuration for ligand binding. In fact, abundance of cysteine residues seems to be one of the common features of the ligand binding domain of many receptors (23). It may be worth noting that the predicted number of amino acids (a.a.) within the extracellular region of the Il-2Rβ chain (214 a.a.) is almost comparable in number to that of the IL-2Rα chain (219 a.a.). Such size similarity may be significant in considering the conformation of the heterodimeric receptor complex that is quite unique for this receptor, as both α and β chains individually interact with distinct sites of the same IL-2 molecule (24).

A hydrophobic stretch of 25 amino acids spanning from the 215 to 239 amino acid residues appears to constitute the membrane spanning region of the receptor (Fig. 1 and 2).

Fig. 2 is a hydropathy plot analysis of deduced human IL-2Rα and Il-2Rβ chain precursor structures. The analysis was carried out according to Kyte and Doolittle (38). SG and TM each represents signal sequence and transmembrane sequence, respectively.

The cytoplasmic region of the β chain consists of 286 a.a. and it is far larger than that of the α chain, which is only 13 a.a. long. The consensus sequences of tyrosine kinase (Gly-x-Gly-x-x-Gly) (25) are absent in the β chain. However, the presence of a triplet, Ala-Pro-Glu (293-295) may be noted; this has been asserted to be the consensus motif for a catalytic domain of some protein kinases (25). The possibility of the cytoplasmic region of the β chain having a protein kinase activity has yet to be tested. The primary structure of this region revealed yet another interesting feature; a rather strong bias for certain characteristic amino acids. This region is rich in proline (42/286) and serine (30/286) residues. Interestingly, the "proline rich" structure has also been demonstrated in the cytoplasmic region of CD2, a T cell membrane antigen involved in the activation pathway of T cells (26). The proline-rich structure may impart a non-globular conformation to this region that may be important in coupling of the receptor molecule with other signal transducer(s). The predominant serine residues may be the major target for phosphorylation, which could also modulate the receptor function (27). In addition, the cytoplasmic region is notably biased for negatively charged amino acids. In fact, this region contains 40 such amino acids (i.e. glutamic and aspartic acids), whereas only 18 amino acids account for the positively charged residues (i.e. lysine and arginine). Such a bias is particularly notable in the middle portion (a.a. 345-390) of the cytoplasmic region. Thus, the cytoplasmic region of the β chain may be quite acidic. Taken together some if not all of these unique characteristics may be responsible in driving further the downstream signal transduction pathway(s). The receptor protein contains 5 potential sites for N-linked glycosylation (Fig. 1), in which 4 are located in the extracellular region. Such a posttranslational modification may account for the difference between the M.W. of the estimated mature (70-75kD) and the calculated (58kD) protein molecules. Hydropathy plot analysis of the α and β chains revealed the presence of hydrophilic regions just adjacent to the cell membrance in the both chains (Fig. 2) These regions may play a role in the non-covalent intramolecular association between the two chains.

According to a one aspect of the present invention therefore we provide a recombinant cDNA coding for an IL-2Rβ chain or a portion thereof.

Preferably a cDNA of the invention has the formula:

GCAGCCAGAGCTCAGCAGGGCCCTGGAGAGATGG
CCACGGTCCCAGCACCGGGGAGGACTGGAGAGCGCGCTGCCACCGCCCC
ATGTCTCAGCCAGGGCTTCCTTCCTCGGCTCCACCCTGTGGATGTA ATG
GCG GCC CCT GCT CTG TCC TGG CGT CTG CCC CTC CTC ATC
CTC CTC CTG CCC CTG GCT ACC TCT TGG GCA TCT GCA GCG
GTG AAT GGC ACT TCC CAG TTC AGA TGC TTC TAC AAC TCG
AGA GCC AAC ATC TCC TGT CTC TGG AGC CAA GAT GGG GCT
CTG CAG GAC ACT TCC TGC CAA GTC CAT GCC TGG CCG GAC
AGA CGG CGG TGG AAC CAA ACC TGT GAG CTG CTC CCC GTG
AGT CAA GCA TCC TGG GCC TGC AAC CTG ATC CTC GGA GCC
CCA GAT TCT CAG AAA CTG ACC ACA GTT GAC ATC GTC ACC
CTG AGG GTG CTG TGC CGT GAG GGG GTG CGA TGG AGG GTG
ATG GCC ATC CAG GAC TTC AAG CCC TTT GAG AAC CTT CGC
CTG ATG GCC CCC ATC TCC CTC CAA GTT GTC CAC GTG GAG
ACC CAC AGA TGG AAC ATA AGC TGG GAA ATC TCC CAA GCC
TCC CAC TAC TTT GAA AGA CAC CTG GAG TTC GAG GCC CGG
ACG CTG TCC CCA GGC CAC ACC TGG GAG GAG GCC CCC CTG
CTG ACT CTC AAG CAG AAG CAG GAA TGG ATC TGC CTG GAG
ACG CTC ACC CCA GAC ACC CAG TAT GAG TTT CAG GTG CGG
GTC AAG CCT CTG CAA GGC GAG TTC ACG ACC TGG AGC CCC
TGG AGC CAG CCC CTG GCC TTC AGG ACA AAG CCT GCA GCC
CTT GGG AAG GAC ACC ATT CCG TGG CTC GGC CAC CTC CTC
GTG GGC CTC AGC GGG GCT TTT GGC TTC ATC ATC TTA GTG
TAC TTG CTG ATC AAC TGC AGG AAC ACC GGG CCA TGG CTG
AAG AAG CTC CTG AAG TGT AAC ACC CCA GAC CCC TCG AAG
TTC TTT TCC CAG CTG AGC TCA GAG CAT GGA GGA GAC GTC
CAG AAG TGG CTC TCT TCG CCC TTC CCC TGA TCG TCC TTC
AGC CCT GGC GGC CTG GCA CCT GAG ATC TCG CCA CTA GAA
GTG CTG GAG AGG GAC AAG GTG ACG CAG CTG CTC CTG CAG
CAG GAC AAG GTG CCT GAG CCC GCA TCC TTA AGC AGC AAC
CAC TCG CTG ACC AGC TGC TTC ACC AAC CAG GGT TAC TTC
TTC TTC CAC CTC CCG GAT GCC TTG GAG ATA GAG GCC TGC
CAG GTG TAC TTT ACT TAC GAC CCC TAC TCA GAG GAA GAC
CCT GAT GAG GGT GTG GCC GGG GCA CCC ACA GGG TCT TCC

CCC CAA CCC CTG CAG CCT CTG TCA GGG GAG GAC GAC GCC TAC TGC ACC TTC CCC TCC AGG GAT GAC CTG CTG CTC TTC TCC CCC AGT CTC CTC GGT GGC CCC AGC CCC CCA AGC ACT GCC CCT GGG GGC AGT GGG GCC GGT GAA GAG AGG ATG CCC CCT TCT TTG CAA GAA AGA GTC CCC AGA GAC TGG GAC CCC CAG CCC CTG GGG CCT CCC ACC CCA GGA GTC CCA GAC CTG GTG GAT TTT CAG CCA CCC CCT GAG CTG GTG CTG CGA GAG GCT GGG GAG GAG GTC CCT GAC GCT GGC CCC AGG GAG GGA GTC AGT TTC CCC TGG TCC AGG CCT CCT GGG CAG GGG GAG TTC AGG GCC CTT AAT GCT CGC CTG CCC CTG AAC ACT GAT GCC TAC TTG TCC CTC CAA GAA CTC CAG GGT CAG GAC CCA ATC CAC TTG GTG TAG ACAGATGGCCAGGGTGGGAGGCAGGCAGCT GCCTGCTCTGCGCCGAGCCTCAGAAGGACCCTGTTGAGGGTCCTCAGTCCA CTGCTGAGGACACTCAGTGTCCAGTTGCAGCTGGACTTCTCCACCCGGATG GCCCCCACCCAGTCCTGCACACTTGGTCCATCCATTTCCAAACCTCCACTG CTGCTCCCGGGTCCTGCTGCCCGAGCCAGGAACTGTGTGTGTTGCAGGGGG GCAGTAACTCCCCAACTCCCTCGTTAATCACAGGATCCCACGAATTTAGGC TCAGAAGCATCGCTCCTCTCCAGCCCTGCAGCTATTCACCAATATCAGTCC TCGCGGCTCTCCAGGGCTCCCTGCCCTGACCTCTTCCCTGGGTTTTCTGCC CCAGCCTCCTCCTTCCCTCCCCTCCCCGTCCACAGGGCAGCCTGAGCGTGC TTTCCAAAACCCAAATATGGCCACGCTCCCCCTCGGTTCAAAACCTTGCAC AGGTCCCACTGCCCTCAGCCCCACTTCTCAGCCTGGTACTTGTACCTCCGG TGTCGTGTGGGGACATCCCCTTCTGCAATCCTCCCTACCGTCCTCCCGAGC CACTCAGAGCTCCCTCACACCCCCTCTGTTGCACATGCTATTCCCTGGGGC TGCTGTGCGCTCCCCCTCATCTAGGTGACAAACTTCCCTGACTCTTCAAGT GCCGGTTTTGCTTCTCCTGGAGGGAAGCACTGCCTCCCTTAATCTGCCAGA AACTTCTAGCGTCAGTGCTGGAGGGAGAAGCTGTCAGGGACCCAGGGCGCC TGGAGAAAGAGGCCCTGTTACTATTCCTTTGGGATCTCTGAGGCCTCAGAG TGCTTGGCTGCTGTATCTTTAATGCTGGGGCCCAAGTAAGGGCACAGATCC CCCCGACAAAGTGGATGCCTGCTGCATCTTCCCACAGTGGCTTCACAGACC CACAAGAGAAGCTGATGGGGAGTAAACCCTGGAGTCCGAGGCCCAGGCAGC AGCCCCGCCTAGTGGTGGGCCCTGATGCTGCCAGGCCTGGGACCTCCCACT GCCCCCTCCACTGGAGGGGTCTCCTCTGCAGCTCAGGGACTGGCACACTGG CCTCCAGAAGGGCAGCTCCACAGGGCAGGGCCTCATTATTTTCACTGCCC CAGACACAGTGCCCAACACCCCGTCGTATACCCTGGATGAACGAATTAATT

ACCTGGCACCACCTCGTCTGGGCTCCCTGCGCCTGACATTCACACAGAGAG
GCAGAGTCCCGTGCCCATTAGGTCTGGCATGCCCCCTCCTGCAAGGGGCTC
AACCCCCTACCCCGACCCCTCCACGTATCTTTCCTAGGCAGATCACGTTGC
AATGGCTCAAACAACATTCCACCCCAGCAGGACAGTGACCCCAGTCCCAGC
TAACTCTGACCTGGGAGCCCTCAGGCACCTGCACTTACAGGCCTTGCTCAC
AGCTGATTGGGCACCTGACCACACGCCCCCACAGGCTCTGACCAGCAGCCT
ATGAGGGGGTTTGGCACCAAGCTCTGTCCAATCAGGTAGGCTGGGCCTGAA
CTAGCCAATCAGATCAACTCTGTCTTGGGCGTTTGAACTCAGGGAGGGAGG
CCCTTGGGAGCAGGTGCTTGTGGACAAGGCTCCACAAGCGTTGAGCCTTGG
AAAGGTAGACAAGCGTTGAGCCACTAAGCAGAGGACCTTGGGTTCCCAATA
CAAAAATACCTACTGCTGAGAGGGCTGCTGACCATTTGGTCAGGATTCCTG
TTGCCTTTATATCCAAAATAAACTCCCCTTTCTTGAGGTTGTCTGAGTCTT
GGGTCTATGCCTTGAAAAAAGCTGAATTATTGGACAGTCTCACCTCCTGCC
ATAGGGTCCTGAATGTTTCAGACCACAAGGGGCTCCACACCTTTGCTGTGT
GTTCTGGGGCAACCTACTAATCCTCTCTGCAAGTCGGTCTCCTTATCCCCC
CAAATGGAAATTGTATTTGCCTTCTCCACTTTGGGAGGCTCCCACTTCTTG
GGAGGGTTACATTTTTTAAGTCTTAATCATTTGTGACATATGTATCTATAC
ATCCGTATCTTTTAATGATCCGTGTGTACCATCTTTGTGATTATTTCCTTA
ATATTTTTTCTTTAAGTCAGTTCATTTTCGTTGAAATACATTTATAAAGAA
AAATCTTTGTTACTCTGTAAATGAAAAAACCCATTTTCGCTATAAATAAAA
GGTAACTGTACAAAATAAGTACAAT

which codes for human Il-2Rβ, or a degenerate variant thereof or a portion thereof or a derivative thereof.

The present invention thus also includes cDNA coding for portions of the complete sequence of the human IL-2Rβ chain for instance the extracellular portion beginning at, or about amino acid (a,a) (see Fig. 1 B) 1 e.g. 1, 2, 3, 4, 5, 6, 7, 8, 9, 10 and ending at or about a.a. 214 e.g. 200, 201, 202, 203, 204, 205, 206, 207, 208, 209, 210, 211, 212, 214, 215, 216, 217, 218, 219, 220, or sub-portions of this extracellular part, or portions corresponding to the intracellular part of the receptor chain e.g. the portion beginning at or about a.a. 239 e.g. a.a. 230, 231, 232, 234, 235 236, 237, 238, 239, 240, 241, 242, up to or about the end a.a. 525, e.g. 516, 517, 518, 519, 520, 521, 522, 523, 524 and 535.

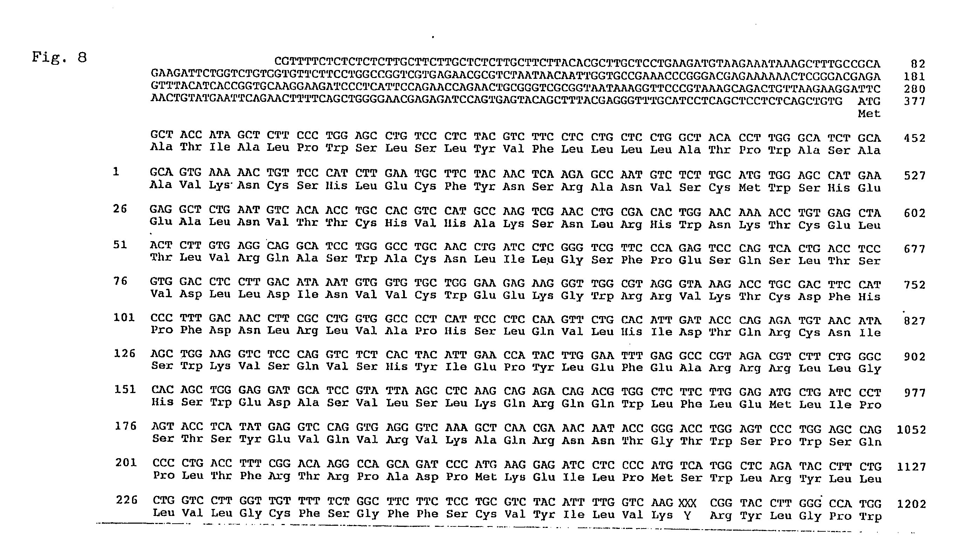

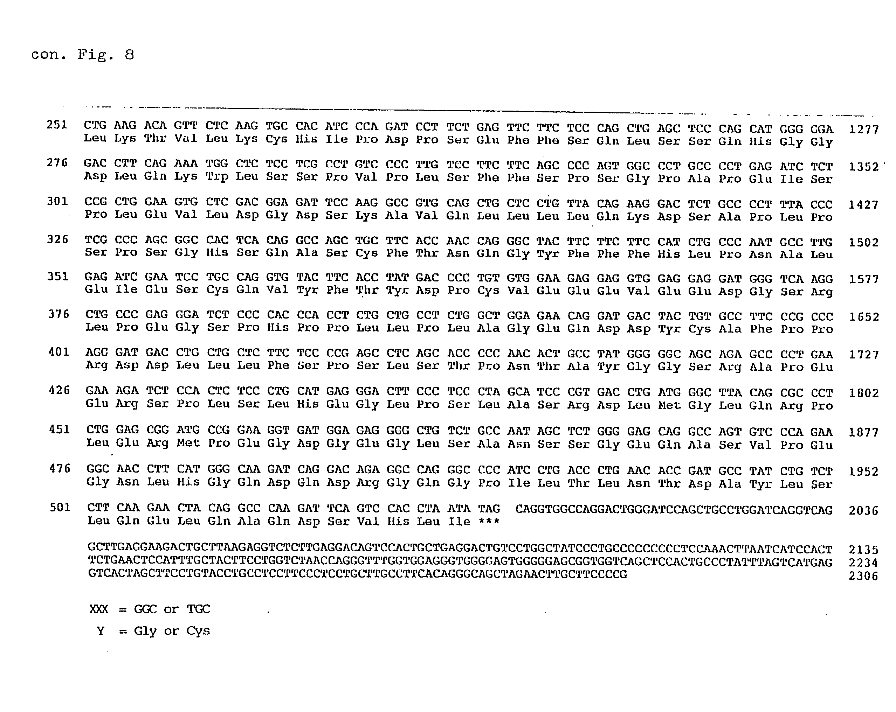

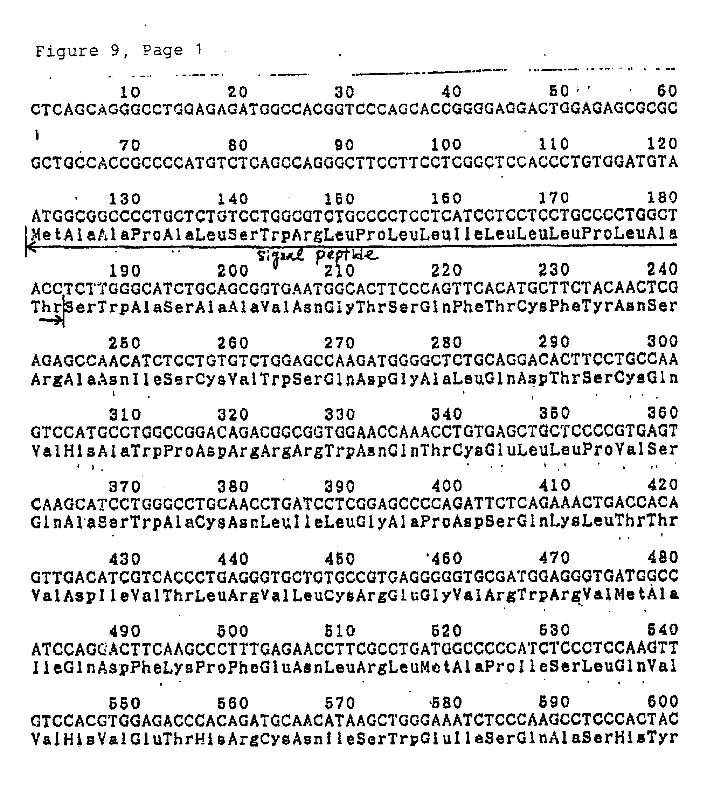

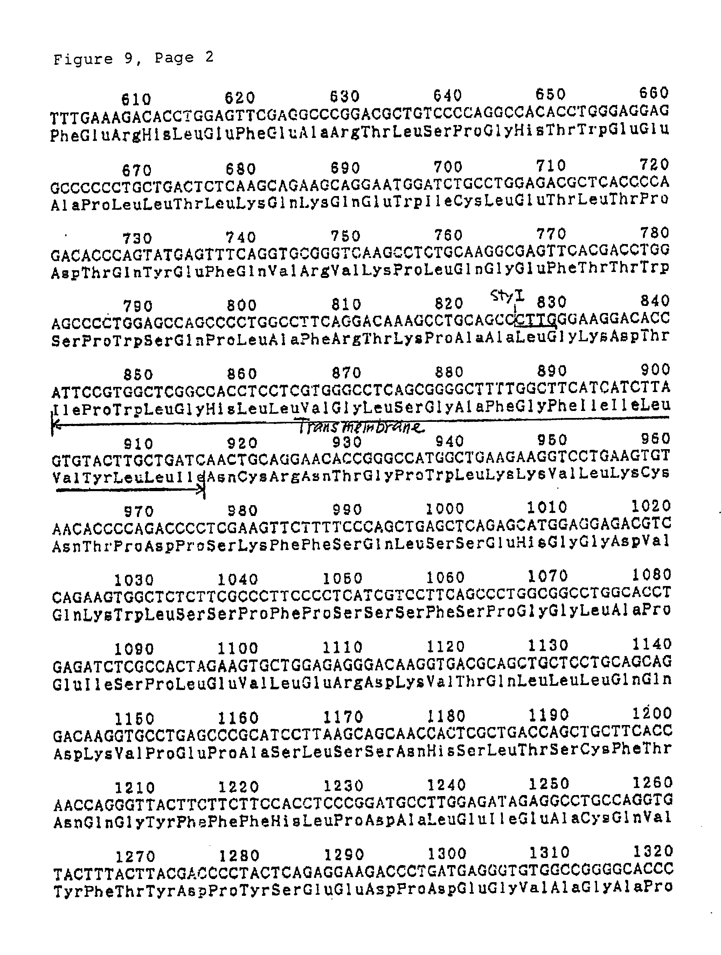

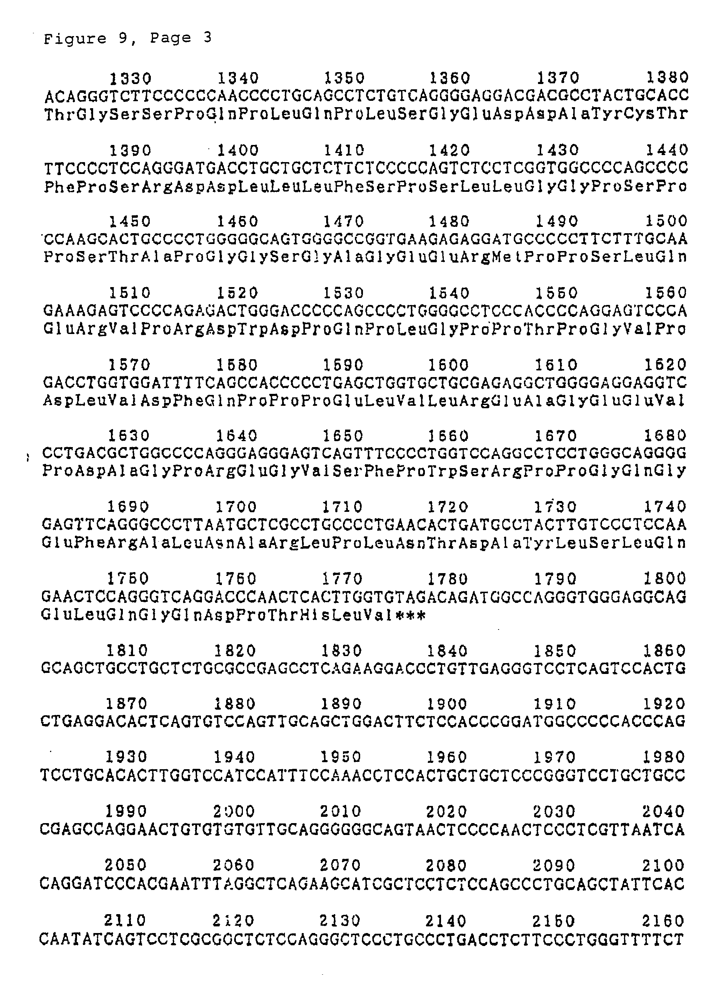

The present invention also includes a recombinant cDNA coding for murine IL-2Rβ chain i.e. the cDNA sequence coding only for amino acids 1 to 513 in Fig. 8 or a degenerate variant therof as well as to the entire neucleotide sequence as set forth in Fig. 8 or a degenerate variant therof, and to cDNA coding for portions of the said sequence, for instance the extracellular part beginnning at, or about amino acid 1 e. g. 1, 2, 3, 4, 5, 6, 7, 8, 9, 10 and ending at or about amino acid 210 e.g. 200, 201, 202, 203, 204, 205, 206, 207, 208, 209, 210, 211, 212, 213, 214, 215, 216, 217, 218, 219, 210 or sub-portions of their extracellular part, or portions corresponding to the intracellular start of the receptor chain e.g. the portion beginning at or about amino acid 235 e.g. amino acid 225, 226, 227, 228, 229, 230, 231, 232, 233, 234, 235, 236, 237, 238, 239, 240, 241, 242, up to or about the end a.a. 513 e.g. 505, 506, 507, 508, 509, 510, 512, and 513.

In one further aspect of the invention we provide a recombinant DNA molecule coding for a water soluble portion of the human IL-2Rβ chain i.e. amino acids about 1 to about 210. Such a cDNA molecule may code, for example, for a soluble human interleukin 2 receptor β-chain derivative having 212 amino acid residues in which residues 1 to 210 correspond to the amino acids of native IL-2Rβ chain.

It will be understood that for the particular IL-2Rβ chains described herein, natural allelic variations exist, occuring from individual to individual. These variations may be demonstrated by one or more amino acid differences in the overall sequence or by deletion substitution insertion, in versions or additions of one or more amino acids in the sequences. In addition it will be understood that the IL-2Rβ chain or portions thereof described herein may be modified by genetic engineering techniques e.g. point mutation, for the substitution, deletion or addition or one or more amino acids without changing the essential characteristics of the IL-2Rβ chain or water soluble part. The present invention thus also includes within its scope cDNA sequences capable of hybridising with the cDNA sequences described herein and coding for proteins having substantially the activity of an IL-2Rβ chain or soluble IL-2Rβ.

For example in one embodiment described below the terminal nucleotides 834 to 848 of a cDNA molecule coding for a soluble IL-2Rβ derivative are as follows

GCC CTT GCT AGC TAG

Ala Leu Ala Ser

Using standard techniques of recombinant DNA technology vectors for transforming suitable host cells can be constructed which contain cDNA sequences corrsponding to the structural gene for IL-2Rβ or any derived portion thereof, or a degenerate variant thereof as set forth above.

Suitable vectors are plasmid vectors for example and will include control and regulatory sequences operably linked to the cDNA sequence coding for the IL-2Rβ chain or portion thereof.

Suitable techniques are well known and widely practised and by way of Example are described, in connection with other proteins in European Patent Applications, Publication Nos. 0254249 and 0170204.

Obtaining the desired portion in pure form from the culture can be carried out by standard techniques and such protein provides a suitable antigen for preparing monoclonal antibodies. Thus hybridomas capable of secreting a monoclonal antibody having a specific affinity to the IL-2Rβ chain or a desired portion thereof may be prepared by immunizing a non-human animal with recombinant IL-2Rβ or a portion thereof, removing spleen cells and fusing these with non-immunoglobulin secreting myelomas cells, and selecting from the resulting hybridomas a cell line which produces a monoclonal antibody having the desired binding specificity and, if desired, subsequently sub-cloning said hybridoma.

The techniques for preparing hybridomas and obtaining monoclonal antibodies in pure form therefrom are well known and by way of example are described in European Patent Application, Publication No. 0168745.

Antibodies in accordance with the invention are useful e.g. for diagnostic purposes and also for therapy by immune suppression or activation. As mentioned above, such antibodies could be raised using purified recombinant protein in accordance with the invention or by transfecting the cDNA of the invention, obtaining cells expressing large amounts of the receptor and using such cells to obtain the antibodies.

The present invention envisages soluble forms of IL-2Rβ chain and of soluble IL-2 receptor. That is the IL-2Rβ chain may be produced in soluble form or the α-chain and β-chain produced simultaneously.

The availability of monoclonal antibodies to specific sub-portions of the IL-2β chain enables epitopes of the receptor chain to be identified and thus opens the way for control of the activity of the receptor to be excerised using suitable monoclonal antibodies or other peptides or peptide mimetic or protein analogue substances.

Expression of Human IL-2Rβ chain mRNA

Expression of the IL-2Rβ mRNA was examined by using the cDNA insert from pIL-2Rβ30 as the probe

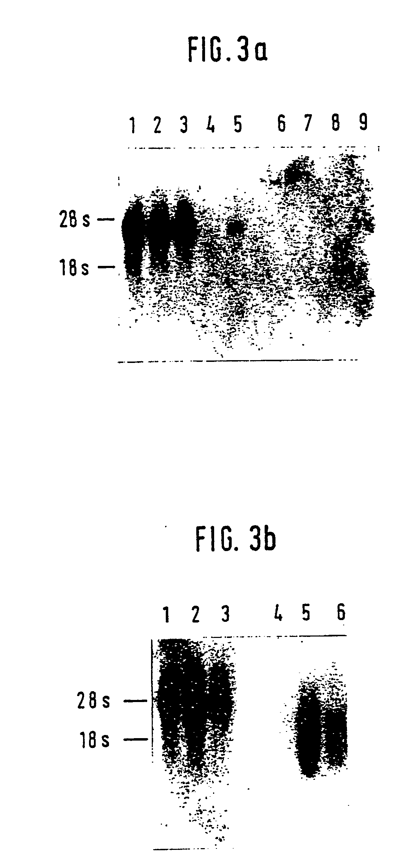

Fig. 3a illustrates the expression of human IL-2Rβ chain mRNA in different cell types. Poly(A)$^+$RNA (2µg per lane) from the following cell sources was prepared and subjected to RNA blotting analysis using the XhoI-digested human IL-2Rβ chain cDNA fragment derived from pIL-2Rβ30 as a probe following standard procedures (14, 18, 27). Lane 1, YT; lane 2, Hut102(HTLV-1 transformed human T cell line); lane 3, MT-2(HTLV-1 transformed human T cell line); lane 4, ARH-77 (multiple myeloma line); lane 5, SKW6.4 (EBV-tranformed human B lymphoblastoid line); lane 6, U937 (histiocytic leukemia line); lane 7, MT-1 (HTLV-1 transformed human T cell line); lane 8, Jurkat (human T leukemic line); lane 9, HeLa (human cervical carcinoma cell line.

As shown in Figure 3a, the RNA blot analysis revealed the presence of a 4kb mRNA, the expression of which is restricted to lymphoid cells previously identified to bear IL-2Rβ chain (i.e. YT, MT-2, Hut102, SKW6.4) (12, 16, 17). On the other hand, the mRNA expression was not detected in cells such as Jurkat, MT-1, U937, ARH-77 and HeLa cells. Essentially, the mRNA expression levels are in correlation with the IL-2Rβ chain expression levels.

Fig. 3 b illustrates the expression of IL-2Rβ and Il-2Rα mRNAs in human PBLs. Total RNA (15µg per lane) was loaded in each lane. Lanes 1 and 4 represent unstimulated human peripheral blood lymphocytes (PBLs); lanes 2 and 5, PBLs stimulated with 5µg/ml phytohemagglutinin (PHA) for 24 hrs; lanes 3 and 6, PBLs stimulated with 5µg/ml PHA for 72 hrs. The RNA-blotted filter was hybridized with the IL-2Rβ probe (lanes 1-3). After dehybridization of the IL-2Rβ probe, the same filter was hybridized with the IL-2Rα probe (XbaI-BclI fragment derived from pSVIL2R-3 (14) (lanes 4-6).

Interestingly, the IL-2Rβ mRNA was detectable in the unstimulated PBLs and its expression levels increased transiently only 2.5-fold after mitogen stimulation. Based on previous data derived from flow cytometric analysis (19), it is likely that the mRNA induction patterns differ between the different lymphocyte populations. This expression pattern is quite different from that of the IL-2Rα chain whose expression strictly requires mitogenic stimulation of the cells (Fig. 3b), suggesting the presence of distinct mechanisms of gene expression between the two genes.

Southern blot analysis of the genomic DNA from PBL and various cell lines including HTLV-1-transformed human T cell lines indicates that the gene is present in a single copy and is not rearranged in those cells.

IL-2 binding properties of the cDNA-encoded IL-2Rβ chain

We next carried out a series of cDNA expression studies in order to examine if the cDNA product binds IL-2 and indeed manifests the properties of the IL-2Rβ chain that have been demonstrated and/or suggested in previous studies. Two cDNA expression plasmids were constructed in which expression of the cDNA spanning the entire coding region was directed by either the mouse lck gene (29) promoter (pLCKRβ) or Moloney leukemia virus LTR (30) (pMLVRβ).

Expression vectors were constructed by the following procedures. pIL-2Rβ30 was digested with HindIII (the cleavage site is located within the polylinker regions of CDM8) and, after fill-in of both ends, a BamHI linker was attached and religated. The resulting plasmid was then digested with BamHI and the 1.8kb DNA fragment which contains the entire coding sequence for the β chain was introduced into BamHI-cleaved p1013 vector containing the mouse lck promoter to construct pLCKRβ. The BamHI-digested cDNA fragment was also introduced into a retrovirus vector, pZipSV(X) (30), to construct pMLVRβ. The human IL-2Rα expressing vector, pSVIL2Rneo, was obtained from pSVIL2R-3 (14) by replacing the Eco-gypt gene with the neo-resistance gene.

The plasmid pLCKRβ was introduced into the mouse T lymphoma EL-4 and the human T cell leukemia Jurkat lines, both of which are known to be devoid of surface molecules that bind human IL-2.

Transfection of the expression plasmids into Jurkat and EL-4 cells was carried out by electroporation as described previously (39). Transfected cells were selected in the RPMI1640 medium containing 10% fetal calf serum (FCS) and G418 (1 mg/ml for EL-4 and 1.5 mg/ml for Jurkat). To obtain cells expressing cDNAs for human IL-2Rα and IL-2Rβ chains simultaneously, a Jurkat-derived clone Jα-5, transfected with pSVIL2Rneo, was co-transfected with pLCKRβ and a plasmid containing the hygromycin-resistance gene, pHgy. The transfected cells were selected with 200µg/ml hygromycin. Transfection of pMLVRβ into Ψ2 cells was carried out by cal- cium-phosphate method as described previously (14) and the cells were selected by 700µg/ml of G418. For flow cytome- tric analysis, $5 \times 10^5$ cells were treated with antibody (1:500 dilution of ascites) at 4° C for 30 min. After was- hing, cells were stained with fluorescein-conjugated goat anti-mouse IgG.

The stained cells were analysed on a FACS440 flow cytometer (Beckton Dickinson). The $^{125}$I-IL-2 binding assay and Scatchard plot analysis were carried out as described previously (12).

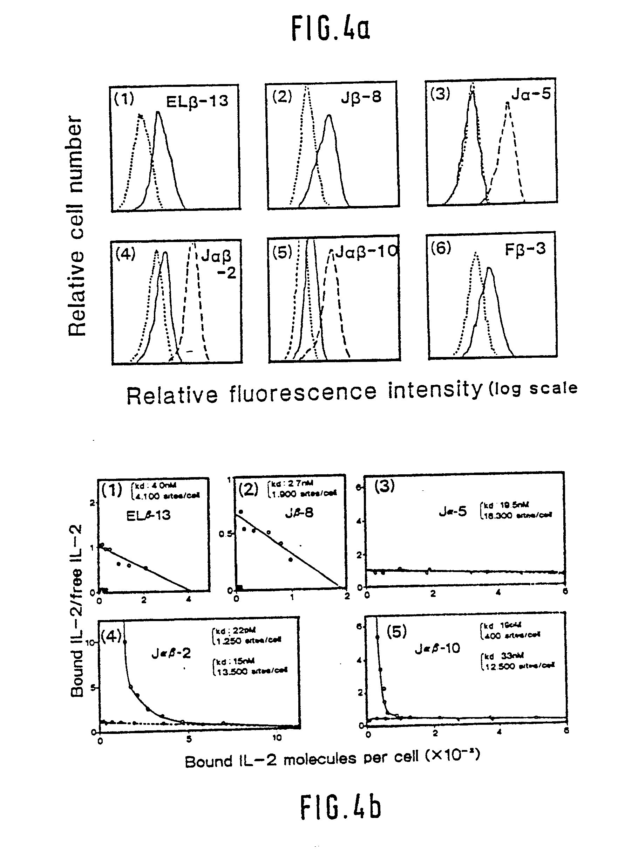

Fig. 4a illustrates the expression of human IL-2Rα and/or IL-2Rβ chain cDNAs by means of cell surface staining patterns of human IL-2Rα and/or IL-2Rβ cDNA transfectants. Parental cells and various transfectant cells were separately stained with either a monoclonal anti-human IL-2Rα antibody, anti-Tac (-----), or monoclonal anti-human IL-2Rβ antibody, Mik-β1 (————), Dotted line (.....) is a fluorescence profile of the cells stained with fluorescein-conjugated goat-anti-mouse IgG alone. Cells used were (1) ELβ-13 (and EL-4-derived clone transfected with pLCKRβ), (2) Jβ-8 (a Jurkat-derived clone transfected with pLCKRβ), (3) α-5 (a Jurkat-derived clone transfected with pSVIL2Rneo), (4) Jα-2 (a Jα-5-derived clone transfected with pLCKRβ), (5) JαB-10 (a Jα-5-derived clone transfected with pLCKRβ), and (6) Fβ-3 (a NIH3T3-derived line transfected with pMLVRβ).

Stable transformant clones expressing the cDNA product were obtained for both the EL-4 (ELβ-13) and Jurkat (Jβ-8 and Jβ-9) cells as judged by FACS analysis (Fig. 4a). In addition, we also introduced the same gene into the Jurkat transformant clone, Jα-5, which expresses the transfected, human IL-2Rα chain cDNA. Two of the resulting transformants, Jαβ-2 and Jαβ-10, were found to express both α and β chains (Fig. 4a-(4), (5)). As expected, RNA blotting analyses of the mRNA expressed in those transformants revealed that the α and β chain-specific mRNAs are derived from the transfected cDNAs but not from the endogeneous genes (26). Furthermore, in order to examine the property of the cDNA product in non-lymphoid cells, the

plasmid pMLVR$\beta$ was intro duced into an NIH3T3 cell-derived cell line 2 (30), and the resulting transformant expressing the cDNA, F$\beta$-3, was obtained (Fig. 4a-(5)).

The IL-2 binding studies were carried out with $^{125}$I-labeled, recombinant human IL-2.

Fig. 4b illustrates the expression of the $\alpha$ and $\beta$ chains by means of the Scatchard plot analysis of $^{125}$I-IL-2 binding to the transfectants expressing the cloned cDNAs. Scatchard plot of the IL-2 binding data in the absence (- - -) or presence (- - -) of 1:100-diluted ascites of Mik-$\beta$1. Binding of $^{125}$I-IL-2 to EL$\beta$-13 or J$\beta$-8 was completely abolished by Mik-$\beta$1. No specific IL-2 binding was observed when parental Jurkat or EL-4 cells were examined. The number of IL-2 binding sites per cell and the receptor affinity were determined by computer-assisted analysis of the IL-2 binding data. (1) EL$\beta$-13, (2) J$\beta$-8, (3) J$\alpha$-5, (4) J$\alpha\beta$-2, (5) J$\alpha\beta$-10.

As can be seen the EL-4-derived clone (EL$\beta$-13) and the Jurkat-derived clone (J$\beta$-8), both expressing the $\beta$ chain cDNA displayed intermediate-affinity to IL-2 with estimated Kd values of 4.0nM and 2.7nM, respectively. The IL-2 binding to those cells was completely abolished by the Mik-$\beta$1 antibody (Fig. 4b-(1), (2)). The Jurkat-derived J$\alpha\beta$-2 and J$\alpha\beta$-10 clones expressing both the human IL-2R$\alpha$ and Il-2R$\beta$ cDNA displayed both high and low affinity receptors with estimated Kp values of 22pM and 15nM for J$\alpha\beta$-2 and 19pM and 33nM for J$\alpha\beta$-10, respectively. In contrast, the parental, Jurkat-derived J$\alpha$-5 cells expressing the $\alpha$ chain cDNA alone manifested exclusively low-affinity (Kd: 19.5nM) to IL-2 (Fig. 4b-(3)). the number of the high-affinity IL-2R expressed J$\alpha\beta$-2 cells and J$\alpha\beta$-10 was comparable to that of expressed IL-2R$\beta$ molecules. In addition, treatment of these cells with Mik-$\beta$1 antibody completely abolished high-affinity IL-2 binding sites from the cell surface, while retaining the expression of low-affinity IL-2R (Fig. 4b-(4), (5)). These observations demonstrate unequivocally that the cDNA-encoded IL-2R$\beta$ molecule is directly involved in the formation of high-affinity receptor complex in association with the IL-2R$\alpha$ chain. In contrast to the aforedescribed T cell transformants, the F$\beta$-3 cells did not display any IL-2 binding on the cell surface under same binding conditions. Interestingly the same observation was made with monkey COS cells that express the $\beta$ chain, but failed to bind IL-2 (28). Thus, the results suggest the involvement of either a cell-type specific processing mechanism(s) or an additional cellular component(s), or both for the functional IL-2R$\beta$ chain expression.

In order to characterize further the molecular structure of reconstituted IL-2R, we performed chemical crosslinking experiments with $^{125}$I-IL-2 and non-cleavable chemical cross-linker, dissuccinimidyl suberate (DSS).

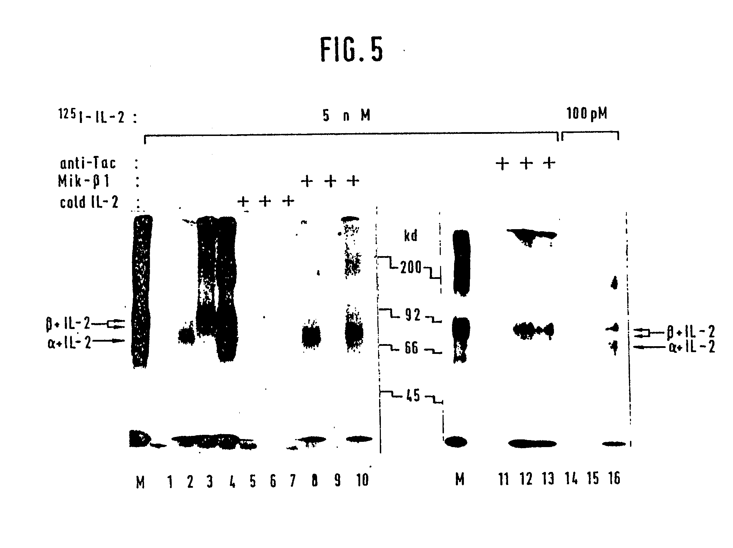

Fig. 5 illustrates the results of the affinity cross-linking studies of the IL-2R-positive transformants. Cells were incubated with 5nM (lanes 1-13) or 100pM (lanes 14-16) of $^{125}$I-IL-2 in the absence (lanes 1-4, 14-16) or presence of a 250-fold molar excess of unlabeled IL-2 (lanes 5-7), 500-fold molar excess of affinity column-purified Mik-$\beta$1 (lanes 8-10) or 500-fold molar excess of affinity column-purified anti-Tac (lanes 11-13). Then cells were chemically crosslinked with dissuccinimidyl suberate (DSS) as described previously (16). The cells were then solubilized and the supernatants were subjected to 7.5% SDS-PAGE. Cells used were: Jurkat (lane 1); J$\alpha$-5 (lanes 2, 5, 8, 11, 14); J$\beta$-8 (lanes 3, 6, 9, 12, 15); J$\alpha\beta$-10 (lanes 4, 7, 10, 13, 16). YT cells crosslinked with $^{125}$I-IL-2 were used as a marker (M).

As can be seen cells expressing only IL-2R$\beta$ chain were crosslinked with $^{125}$I-labeled IL-2 and analysed by SDS-PAGE, a doublet band consisting of 90kD major and 85kD minor was detected and its migration profile was indistinguishable from that of YT cells (see arrows in Fig. 5 and ref. 16, 17). The appearance of the doublet is inhibited by an excess of unlabeled IL-2 or by Mik-$\beta$1. The doublet formation may be due to degradation of receptor-IL-2 complex. It is also possible that both protein products are derived by a differential post-translational modification(s). Alternatively, one of the doublet may represent a third component of the receptor complex. A broad band migrating around the position of 150kD was also detected in the transfectant (J$\alpha\beta$-10) as well as YT cells. The appearance of this band is also inhibited by either unlabeled IL-2 or Mik-$\beta$1. It may represent the ternary complex of IL-2, IL-2R$\alpha$ and Il-2R$\beta$ molecules. In a series of chemical cross-linking experiments shown in Fig. 4, it was demonstrated that the physico-chemical properties of the receptor complex expressed on the surface of J$\alpha\beta$-2 are indistinguishable from the properties of high-affinity receptor expressed on cultured T cells or PBLs (12, 16, 17).

Preliminary results of experiments to determine whether the expression of the $\alpha$ and $\beta$ chains in non-lymphoid cells results in the formation of high-affinity receptor indicate that, when the $\alpha$ and $\beta$ chain cDNAs are co-expressed transiently in COS cells, both chains can crosslink with $^{125}$I-IL-2 at the concentration (400 pM) in which the similarly expressed $\alpha$ chain alone can not (28). The results may suggest the formation of the $\alpha\beta$ heterodimeric receptor in this non-lymphoid cell line.

IL-2 internalization by reconstituted receptors

It has been reported that intermediate- and high-affinity IL-2 receptors can both internalize IL-2 (33-35). Ligand internalization is usually accompanied with the IL-2 signal transduction, suggesting this process to be essential.

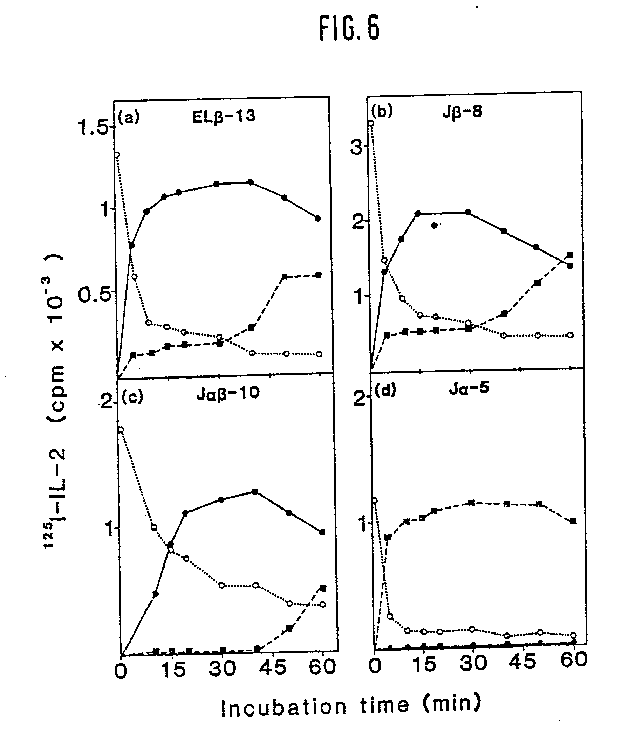

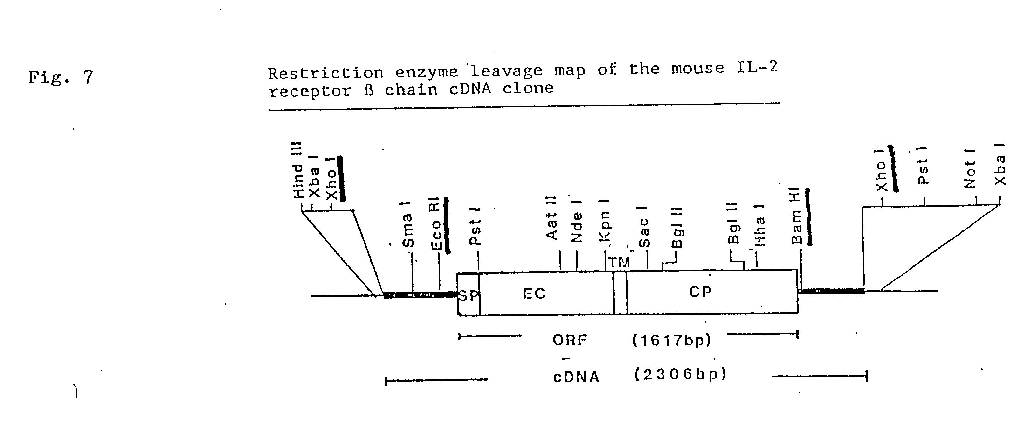

Fig. 6 illustrates IL-2 internalization via the reconstituted receptors. IL-2 internalization was examined according to a method described previously (33). Briefly, cells ($5x10^7$) were treated with $^{125}$I-IL-2 at a final concentration of 200pM (Jαβ-10) or 5nM (Jα-5, Jβ-8 and ELβ-13) at 0° C for 30 min. After washing, cells were suspended with prewarmed culture medium (37° C) and the kinetics of IL-2 internalization was examined as described previously (33). (a) ELβ-13, (b) Jβ-8, (c) Jαβ-10, (d) Jα-5. (-●-●-●-), internalized IL-2; (...O...O...), cell-surface bound IL-2; (-■-■-■-■-), free IL-2.

As shown in Fig. 6, we examined whether the reconstituted receptors can internalize IL-2. In fact, the cells expressing IL-2Rβ chain alone, or both α and β chains are capable of internalizing IL-2 following a kinetic pattern similar to that reported for the native receptor. In contrast, the Jurkat cells expressing only IL-2Rα failed to internalize IL-2, similar to previously reported observations (33, 34). Preliminary results indicate that the growth of the cells expressing the intermediate- or high-affinity receptors is selectively inhibited by IL-2 (14, 36). We also have preliminary results that the β chain expressed in another host cell line functions in stimulating the cell growth in response to IL-2 (28).

## Cloning of Murine IL-2R receptor β chain

No specific antibodies to murine IL-2 receptor β-chain are known to exist, accordingly the screening method used for the isolation of cDNA for Hu IL-2Rβ chain was not employed.

A cDNA library was prepared using poly $(A)^+$-RNA from Concanavalin A stimulated mouse spleen cells; the cDNA was cloned in λgt 10 which was multiplied in E. coli.

Screening of this library was then carried out using the above described human IL-2Rβ chain cDNA as the probe under non-stringent conditions. From the positive clones a clone designated λMIL 2Rβ-26 was selected. The cDNA insert in this clone contained only a 540 bp sequence of the whole murine Il-2Rβ chain sequence. This sequence was therefore isolated by digestion of λMIL-2Rβ-26 using Pvu 2 and used for screening another cDNA library prepared using poly $(A)^+$ from the mouse thymoma cell line EL-4 according to standard procedures and cloned into the BstXI site of the CDM8 vector followed by transfecting E. coli.

Screening of the cDNA library was carried out under highly stringent conditions according to the method described in European Patent Application No. 88 119 602.9 and Kashima et al. (Nature Vo. 313 pp 402-404, 1985).

From the positive clones clone pMIL-2Rβ-36 containing the structural gene for murine IL-2Rβ was selected.

Plasmid pMIL-2Rβ-36 has been deposited in strain E. coli MC 1061/P3 on May 23, 1989 at the Fermantation Research Institute according the Budapest Treaty under accession number FERM BP-2435.

## Preparation of soluble human interleukin 2 receptor β-chain

A secreted form of the hIL2-Rβ chain (termed hereafter soluble β) was produced by transfecting NIH 3T3 fibroblasts with the modified β-chain cDNA ("anchor minus" cDNA) lacking the entire DNA sequence encoding both intracytoplasmic and transmembrane domains of the native β-chain.

### Construction of the expression vector harboring the anchor minus cDNA which encodes the soluble β - (BCMGNeo-sol.β)

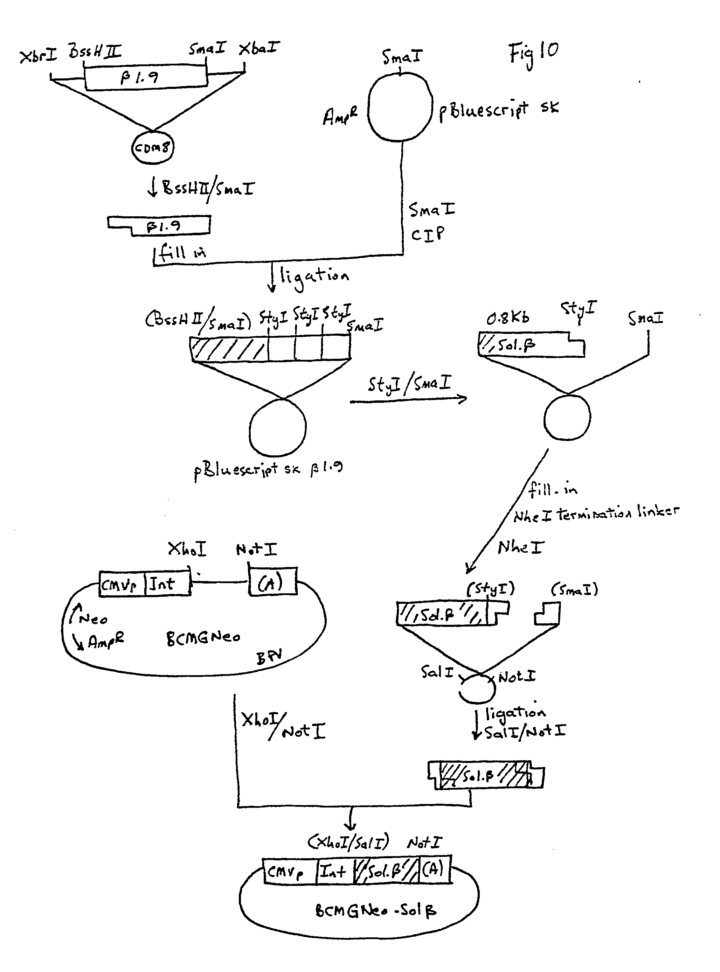

The β-chain cDNA (Fig. 1b) was modified into the anchor minus form for the production of the soluble β. A strategy to generate the expression vector containing the anchor minus cDNA is illustrated in Fig. 10. First, the plasmid pIL-2Rβ30 containing the 2.3-kb β-chain cDNA in the CDM8 vector was digested with BssH II and Sma I (all restriction enzymes were purchased from New England BioLabs, Beverly, MA, USA), and a 1.9-kb cDNA fragment (base 58-1967) including the entire coding sequence (base 121-1773) of the β-chain was obtained. After fill-in at the BssH II end, the 1.9-kb cDNA was inserted into a Sma I restriction site of pBluescript SK vector (Stratagene, San Diego, CA, USA). This pBluescript SK-β1.9 plasmid was then digested with Sty I (restriction sites; base 825, 934 and 1235) and Sma I so that all of the intracytoplasmic and transmembrane regions were deleted, leaving bases 121-840 representing most of the extracellular region intact. Next, a 12-base synthetic linker (New England BioLabs, #1060) containing multiple termination codons (TAG) as well as the recognition sequence for Nhe I was phosphorylated and ligated to the Sty I/Sma I-digested plasmid DNA with T4 DNA ligase. After digestion with the Nhe I to remove excess linker, the DNA was ligated to the SK vector to construct pBluescript SK-sol.β.

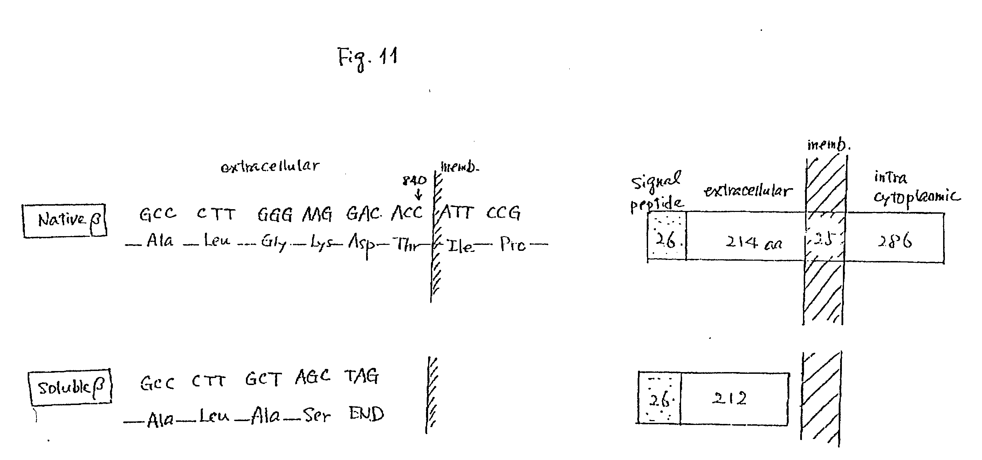

This pBluescript SK-sol.β was digested with Sal I and Not I (the restriction sites were located within the polylinker region of the pBluescript SK vector), and the resulting 0.8-kb cDNA fragment encoding the soluble β was isolated. This cDNA fragment was introduced into Xho I/Not I-digested BCMGNeo vector (see Karasuyama et al., J. Exp. Med. 169: 13-25, 1989) containing the cytomegalovirus (CMV) promoter and neomycin-resistance gene to generate the final expression plasmid BCMGNeo-sol.β. The BCMGNeo is a shuttle vector containing 69 % of bovine papilloma virus (BPV) sequences which ensure extrachromosomal replication in mammalian cells. As illustrated in Fig. 11, which illustrates the nucleotide sequence and corresponding amino acid sequence for the native and soluble β, the soluble β cDNA encodes a mature protein consisting of 212 amino acids (aa) accompanied by a signal peptide of 26 aa, while the native β-chain cDNA encodes a membrane protein consisting of a signal peptide (26 aa), extracellular (214 aa), transmembrane (25 aa), and intracytoplasmic (286 aa) domains. The nucleotide and corresponding amino acid sequences for the native and soluble β are also illustrated in Fig. 11.

Transfection of NIH 3T3 fibroblast with BCMGNeo-sol.β and establishment of stable transformants secreting the soluble β

cDNA transfection was performed by the protoplast fusion technique as described in Karasuyama et al. (supra.). Briefly, bacteria, containing the BCMGNeo-sol.β were converted to protoplasts and fused with a murine fibroblast cell line, NIH 3T3 by using polyethylene glycol 2,000 (Wako Chemical Industries, Osaka, Japan). Ten million protoplast-fused NIH 3T3 cells were then seeded in four 24-well plates. Twenty five days after the culture in RPMI 1640 medium containing 10 % fetal calf serum (FCS) and 750 µg/ml of G418 (Geneticin; Sigma, St. Louis, MO, USA), transformant-cell growth was observed in 60 wells out of 104. When determined by the sandwich enzyme-linked immunosorbent assay (ELISA) as described below, culture supernatants from 18 wells out of 60 were found to be positive for the soluble β. Five clones were established by limiting dilution from a well which gave the highest absorbance in the ELISA, and they were all found to secret high levels of soluble β (Table I). In contrast, NIH 3T3 cells transfected with the full-length β-chain cDNA (designated 3T3-β11) did not secrete the β-chain molecule to any extent. In the subsequent studies, we used the clone designated 3T3-B4-14 which secreted the highest amount of the soluble β.

Table 1

Levels of the soluble β in the culture supernatant of NIH 3T3 fibroblats transfected with BCMGNeo-sol.β

| Culture supernatant | Absorbance at 405 nm in ELISA |
| --- | --- |
| 3T3-B4-1 | 1.492 |
| 3T3-B4-4 | 1.301 |
| 3T3-B4-7 | 1.259 |
| 3T3-B4-14 | 1.579 |
| 3T3-B4-19 | 1.533 |
| 3T3-β11 | 0.052 |
| medium alone | 0.072 |

ELISA for detecting the soluble β

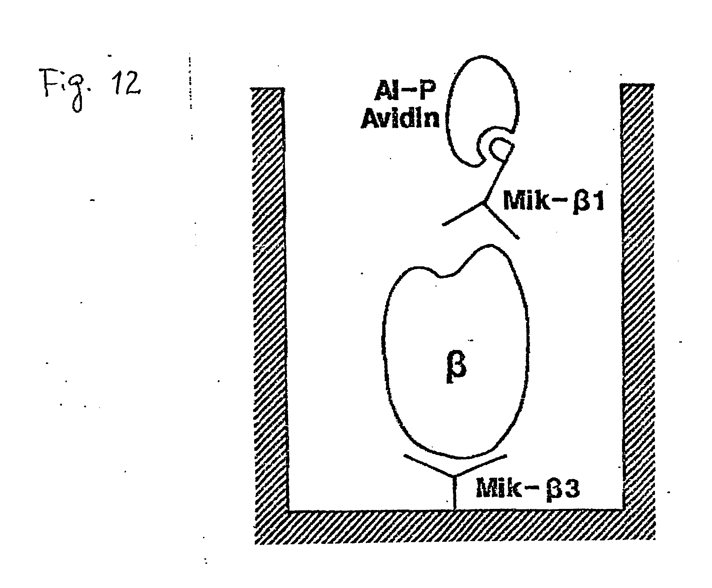

Culture supernatants of transfected cells were screened for the presence of the soluble β by a sandwich enzyme-linked immunosorbent assay (ELISA). In this assay were used two monoclonal antibodies Mik-β1 and -β3, supra. and Tsudo et al., Proc. Natl. Acad.Sci. USA, 86: 1982-1986, 1989, which recognize the distinct epitopes on the β-chain; i.e. Mik-β1 recognises the IL-2 binding site, while Mik-β3 recognises the epitope not involved in the IL-2 binding. As illustrated in Fig. 12, which is a schematic representation of the Sandwich ELISA Immulon-I microtiter plates (Dynatec, Chantilly, VA, USA) were coated overnight with 50 µl of Mik-β3 at 10 µg/ml in Tris-buffered saline (10 mM Tris-HCl, pH 7,4, 0,15 M NaCl). After discarding excess antibody, unbound sites were blocked by incubating with TBS containing 1 % bovine serum albumin for 1 hour. After washing with TBS containing 0,05 % Tween 20 (T-TBS), 50 µl of culture supernatants of the transformants were added to the wells and incubated for 1 hour. After washing, 50 µl of biotinylated Mik-β1 at 1 µg/ml were added as the secondary antibody to detect the soluble β bound to the primary antibody, Mik-β3 on the plate. After 45 minutes incubation and subsequent washing, 50 µl of alkaline phosphatase-conjugated avidin (Tago, Burlingame, CA, USA) were added. After a 45 minutes incuba- tion, the plates were washed, 100 µl of p-nitrophenyl phosphate were added, and the absorbance of the wells was determined at 405 nm after 45 minutes.

Apparent molecular weight of secreted soluble β

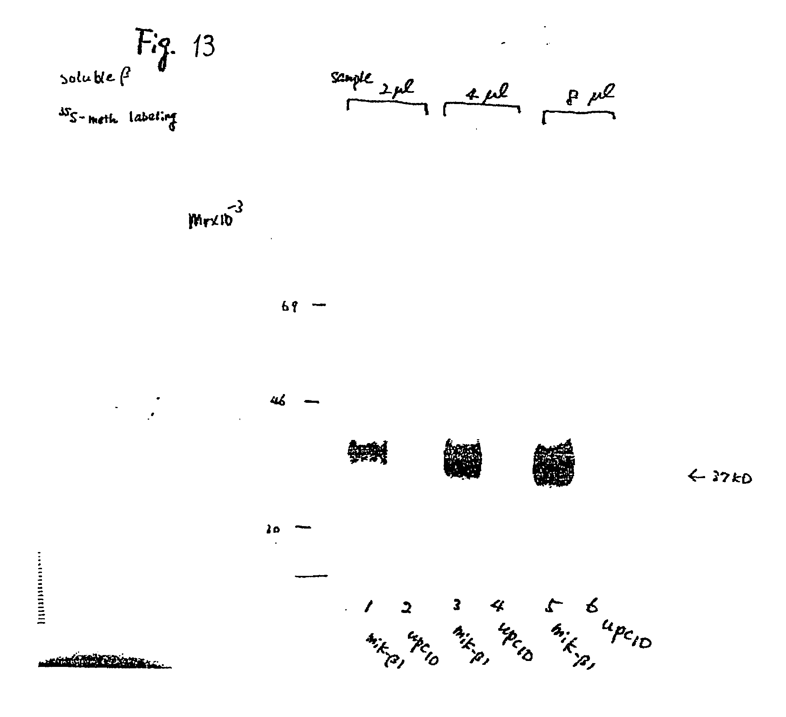

In order to define the molecular size of the soluble β, the 3T3-B4-14 cells were biosynthetically labeled with $^{35}$S-methionine, and the soluble β immunoprecipitated by the Mik-β1 mAb from the culture supernatant. Various amounts of immuno precipitates using the Mik-β1 and, as control UPC 10mAb for precipitation were loaded and electrophoresed on an 8 % SDS-polyacrylamide gel. As shown in Fig. 13, when examined by the SDS-polyacrylamide gel electrophoresis (PAGE), the Mik-β1, but not the control UPC 10 mAb, identified a single species of protein with an apparent Mr of 37,000 in the culture supernatant of 3T3-B4-14 cells. This molecular size is in good agreement with that predicted for the truncated β-chain lacking all of the transmembrane (25 aa) and intracytoplasmic (286 aa) regions.

IL-2 binding ability of the soluble β

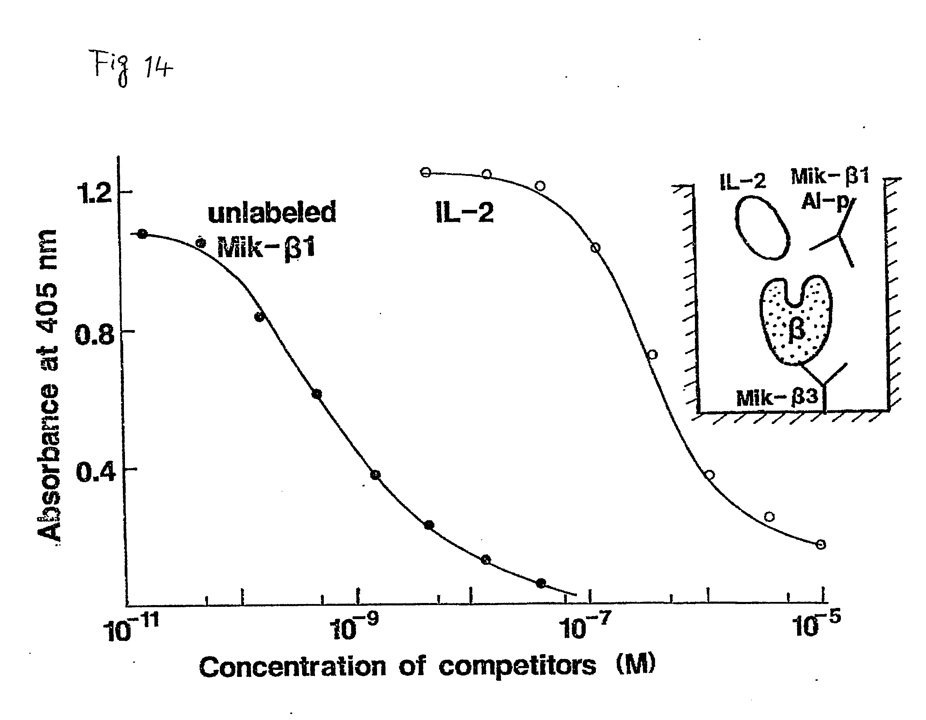

It was then investigated whether the secreted form of the β-chain is capable of binding IL-2. To this end, a "competitive" sandwich ELISA was employed. In this assay, the soluble β in the culture supernatant was fixed on the solid phase by the Mik-β3 mAb, a non-inhibitory mAb for IL-2 binding, so that the putative IL-2 binding site on the β-chain would remain unoccupied. Then, serial dilutions of IL-2 or unlabeled Mik-β1 were added as competitors for biotinylated Mik-β1. Fig. 14 shows the results of the competitive sandwich ELISA in which the curve -●-●- is for serial dilutions with unlabeled Mik-β1 and -O-O- for serial dilutions of IL-2. As shown in Fig. 14 unlabeled Mik-β1 reduced the absorbance dose-dependently, showing the specificity of this system. Likewise, IL-2 efficiently competed dose-dependently with biotinylated Mik-β1 for the binding to the soluble β, indicating that the soluble β is indeed capable of binding IL-2.

This competition curve is quite similar to that found for the detergent-solubilised native β-chain from YTS cells which express the β-chain alone, indicating that the affinity of the soluble β to IL-2 is comparable to that of the solubilised native β-chain.

The availability of the genes encoding IL-2Rβ chains makes it possible to explore novel approaches for the functional studies of the IL-2 system. The receptor structure operating in the IL-2 system is unique in that two structurally distinct membrane molecules, the IL-2Rα and IL-2Rβ chains, both bind IL-2 independently. The series of cDNA expression examples described herein substantiate further the previous notion that the α and β chains constitute the high-affinity IL-2R complex via a non-covalent association of the molecules (18, 37). Thus the peculiarity of this system is ,the involvement of three intermolecular interactions between one ligand and two distinct receptors. By virtue of the present invention it will now be possible to elucidate functional domains of this unique cytokine receptor system. Mutational analyses of the cloned β chain cDNA may provide clues as to the identification of respective domains involved in ligand binding and association with the α chain. To date, little is known about the cascade of biochemical events triggered by cytokines interacting with their homologous receptors. By the present invention we have demonstrate the presence in the IL-2Rβ chain of a large cytoplasmic region which most likely is involved in driving the IL-2 signal pathway(s). The particular acidic nuclei found in the cytoplasmic region may suggest coupling to other cytoplasmic signal transducers. Alternatively, in view of a previous report on the presence of IL-2 within the nucleous (33), an intriguing possibility is that the acidic as well as the proline-rich regions of the IL-2Rβ cytoplasmic component may play a role in activation of the genetic programming. The availability of the expression system in which the cDNA-encoded β chain can deliver growth signals will allow further clarification of the functional domaines of the receptor. It is now possible to study the essential role of IL-2 in the development and regulation of the immune system.

The availability of soluble counterparts to the cell surface receptor β-chain should facilitate structural analysis of the β-chain since crystallisation of soluble molecules is more easily accomplished than insoluble ones. The soluble molecules can also be used to neutralise the actual cell surface receptors for studies of the biological functions of the receptors or for therapeutic purposes.

REFERENCES AND NOTES

1.D.A. Morgan, F.W. Ruscelli, R.C. Gallo, Science 198, 1007 (1976)
2. K.A. Smith, Annu.Rev.Immunol. 2, 319 (1984); T. Taniguchi et al., Immunol.Rev. 92, 121 (1986)
3. D.H. Raulet, Nature 314, 101 (1985)
4. M. Tsudo, T. Uchiyama, H. Uchino, J.Exp.Med. 160, 612 (1984); T.A. Waldmann et al., ibid., p. 1450 (1984); M.A. Blackman, M.A. Tigges, M.E. Minis, M.E. Koshland, Cell, 47, 609 (1986)
5. M. Malkovsky et al., Nature 325, 262 (1987)
6. C.S. Henney, K. Kuribayashi, D.E. Kern, S. Gillis, ibid. 291, 335 (1981)
7. M.E. Lotze, E.A. Grimm, S.A. Strausser, S.A. Rosenberg, Cancer Res. 41, 4420 (1981); E.A. Grimm, A. Mazumder, H.Z. Zhang, S.A. Rosenberg, J.Exp. Med. 155, 1823 (1982
8. S.A. Rosenberg et al., N.Engl.J.Med. 316, 889 (1987)
9. E.N. Benveniste, J.E. Merrill, Nature 321, 610 (1986)
10. S.J. LeGrue, Lymphokine Res. 7, 1987 (1988); G.B. Millis, P. Girard, S. Grinstein, E.W. Gelfand, Cell 55, 91 (1988); V.E. Valge, J.G.P. Wong, B.M. Datlof, A.J. Sinskey, A. Rao, ibid., p. 101 (1988); M.A. Tigges, L.S. Casey, M.E. Koshland, Science 243, 781 (1989)
11. R.J. Robb, W.C. Greene, C.M. Rusk, J.Exp.Med. 160, 1126 (1984)
12. M. Tsudo, R.W. Kozak, C.K. Goldman, T.A. Waldmann, Proc.Natl.Acad.Sci.U.S.A. 83, 9694 (1986); K. Teshigawara, H.-M. Wang, K.Kato, K.A. Smith, J.Exp.Med. 165, 223 (1987)
13. W.J. Leonard et al., Nature 311, 626 (1984) T. Nikaido et al., ibid., p. 631 (1984); D. Cosman et al., ibid. 312, 768 (1984)
14. M. Hatakeyama et al., ibid. 318, 467 (1985)
15. W.C. Greene et al., J. Exp. Med. 162, 363 (1985), S. Kondo et al., ibid. 320, 75 (1986); R.J. Robb, Proc.Natl. Acad. Sci.U.S.A. 83, 3992 (1986)
16. M. Sharon, R.D. Klausner, B.R. Cullen, R. Chizzonite, W.J. Leonard, Science 234, 859 (1986)
17. R.J. Robb et al., Proc.Natl.Acad.Sci.U.S.A., 84, 2002 (1987); M. Tsudo, R.W. Kozak, C.K. Goldman, T.A. Waldmann, ibid., p 4215 (1987); M. Dukovich et al., Nature 327, 518 (1987)
18. M. Hatakeyama et al., J.Exp.Med. 166, 362 (1987); S. Kondo et al., Nature 327, 64 (1987)
19. M. Tsudo, F. Kitamura, M. Miyasaka, Proc.Natl.Acad.Sci.U.S.A. in press.
20. J. Yodoi et al., J.Immunol. 134, 1623 (1985)
21. B. Seed, A. Aruffo, Proc.Natl.Acad.Sci.U.S.A. 84, 3365 (1987); B. Seed, Nature 328, 840 (1987)
22. D. Perlman, H.O. Halvorson, J.Mol.Biol. 167, 391 (1983); G. von Heijne, Nucleic Acids Res. 14, 4683 (1986)
23. A. Ullrich et al., Nature 309, 418 (1984); A. Ullrich et al., ibid. 313, 756 (1985); U. Ebina et al., Cell 40, 747 (1985)
24. L. Collins et al., Proc.Natl.Acad.Sci.U.S.A. 85, 7709 (1988)
25. K.S. Hanks, A.M. Quinn, T. Hunter, Science 241, 42 (1988)
26. A. Alcover et al., Immunol.Rev. 95,5 (1987)
27. M. Hatakeyama, S. Minamoto, T. Taniguchi, Proc.Natl.Acad.Sci.U.S.A. 83, 9650 (1986)
28. M. Hatakeyama, T. Doi, T. Kono, S. Minamoto, T. Taniguchi, unpublished observations
29. J.D. Marth, R. Peet, E.G. Krebs, R.M. Perlmutter, Cell 43, 393 (1985)
30. R. Mann, R.C. Mulligan, D. Baltimore, ibid. 33, 153 (1983)
31. M. Fujii et al., J.Exp.Med. 163, 550 (1986)
32. A.M. Weissman et al., Proc.Natl.Acad.Sci.U.S.A. 83, 1463 (1986)
33. R.J. Robb, W.C. Greene, J.Exp.Med. 165, 1201 (1987)
34. K. Sugamura et l., ibid. 161, 1243 (1985)
35. H. Saragovi, T.R. Malek, J.Immunol. 141, 476 (1988)
36. J. Kyte, R.F. Doolittle, J.Mol.Biol. 157, 105 (1982)
37. H. Potter, L. Weir, P. Leder, Proc.Natl.Acad.Sci.U.S.A. 81, 7161 (1984)

## Claims

1. A recombinant DNA molecule coding for the β-chain of an IL-2 receptor or a portion thereof.

2. A recombinant DNA molecule as defined in claim 1 coding for the β-chain of human or murine IL-2 receptor or a portion thereof.

3. A recombinant DNA molecule characterized by a structural gene having the formula:

```
                                                       ATG
GCG GCC CCT GCT CTG TCC TGG CGT CTG CCC CTC CTC ATC
CTC CTC CTG CCC CTG GCT ACC TCT TGG GCA TCT GCA GCG
GTG AAT GGC ACT TCC CAG TTC AGA TGC TTC TAC AAC TCG
AGA GCC AAC ATC TCC TGT CTC TGG AGC CAA GAT GGG GCT
CTG CAG GAC ACT TCC TGC CAA GTC CAT GCC TGG CCG GAC
AGA CGG CGG TGG AAC CAA ACC TGT GAG CTG CTC CCC GTG
AGT CAA GCA TCC TGG GCC TGC AAC CTG ATC CTC GGA GCC
CCA GAT TCT CAG AAA CTG ACC ACA GTT GAC ATC GTC ACC
CTG AGG GTG CTG TGC CGT GAG GGG GTG CGA TGG AGG GTG
ATG GCC ATC CAG GAC TTC AAG CCC TTT GAG AAC CTT CGC
CTG ATG GCC CCC ATC TCC CTC CAA GTT GTC CAC GTG GAG
ACC CAC AGA TGG AAC ATA AGC TGG GAA ATC TCC CAA GCC
TCC CAC TAC TTT GAA AGA CAC CTG GAG TTC GAG GCC CGG
ACG CTG TCC CCA GGC CAC ACC TGG GAG GAG GCC CCC CTG
CTG ACT CTC AAG CAG AAG CAG GAA TGG ATC TGC CTG GAG
ACG CTC ACC CCA GAC ACC CAG TAT GAG TTT CAG GTG CGG
GTC AAG CCT CTG CAA GGC GAG TTC ACG ACC TGG AGC CCC
TGG AGC CAG CCC CTG GCC TTC AGG ACA AAG CCT GCA GCC
CTT GGG AAG GAC ACC ATT CCG TGG CTC GGC CAC CTC CTC
GTG GGC CTC AGC GGG GCT TTT GGC TTC ATC ATC TTA GTG
TAC TTG CTG ATC AAC TGC AGG AAC ACC GGG CCA TGG CTG
```

AAG AAG CTC CTG AAG TGT AAC ACC CCA GAC CCC TCG AAG
TTC TTT TCC CAG CTG AGC TCA GAG CAT GGA GGA GAC GTC
CAG AAG TGG CTC TCT TCG CCC TTC CCC TGA TCG TCC TTC
AGC CCT GGC GGC CTG GCA CCT GAG ATC TCG CCA CTA GAA
GTG CTG GAG AGG GAC AAG GTG ACG CAG CTG CTC CTG CAG
CAG GAC AAG GTG CCT GAG CCC GCA TCC TTA AGC AGC AAC
CAC TCG CTG ACC AGC TGC TTC ACC AAC CAG GGT TAC TTC
TTC TTC CAC CTC CCG GAT GCC TTG GAG ATA GAG GCC TGC
CAG GTG TAC TTT ACT TAC GAC CCC TAC TCA GAG GAA GAC
CCT GAT GAG GGT GTG GCC GGG GCA CCC ACA GGG TCT TCC
CCC CAA CCC CTG CAG CCT CTG TCA GGG GAG GAC GAC GCC
TAC TGC ACC TTC CCC TCC AGG GAT GAC CTG CTG CTC TTC
TCC CCC AGT CTC CTC GGT GGC CCC AGC CCC CCA AGC ACT
GCC CCT GGG GGC AGT GGG GCC GGT GAA GAG AGG ATG CCC
CCT TCT TTG CAA GAA AGA GTC CCC AGA GAC TGG GAC CCC
CAG CCC CTG GGG CCT CCC ACC CCA GGA GTC CCA GAC CTG
GTG GAT TTT CAG CCA CCC CCT GAG CTG GTG CTG CGA GAG
GCT GGG GAG GAG GTC CCT GAC GCT GGC CCC AGG GAG GGA
GTC AGT TTC CCC TGG TCC AGG CCT CCT GGG CAG GGG GAG
TTC AGG GCC CTT AAT GCT CGC CTG CCC CTG AAC ACT GAT
GCC TAC TTG TCC CTC CAA GAA CTC CAG GGT CAG GAC CCA
ATC CAC TTG GTG TAG

or a portion thereof or a degenerate variant thereof.

4. A recombinant DNA molecule according to claim 3 characterized by a DNA sequence having the formula:

GCAGCCAGAGCTCAGCAGGGCCCTGGAGAGATGG
CCACGGTCCCAGCACCGGGGAGGACTGGAGAGCGCGCGCTGCCACCGCCCC
ATGTCTCAGCCAGGGCTTCCTTCCTCGGCTCCACCCTGTGGATGTA ATG
GCG GCC CCT GCT CTG TCC TGG CGT CTG CCC CTC CTC ATC
CTC CTC CTG CCC CTG GCT ACC TCT TGG GCA TCT GCA GCG
GTG AAT GGC ACT TCC CAG TTC AGA TGC TTC TAC AAC TCG

AGA GCC AAC ATC TCC TGT CTC TGG AGC CAA GAT GGG GCT
CTG CAG GAC ACT TCC TGC CAA GTC CAT GCC TGG CCG GAC
AGA CGG CGG TGG AAC CAA ACC TGT GAG CTG CTC CCC GTG
AGT CAA GCA TCC TGG GCC TGC AAC CTG ATC CTC GGA GCC
CCA GAT TCT CAG AAA CTG ACC ACA GTT GAC ATC GTC ACC
CTG AGG GTG CTG TGC CGT GAG GGG GTG CGA TGG AGG GTG
ATG GCC ATC CAG GAC TTC AAG CCC TTT GAG AAC CTT CGC
CTG ATG GCC CCC ATC TCC CTC CAA GTT GTC CAC GTG GAG
ACC CAC AGA TGG AAC ATA AGC TGG GAA ATC TCC CAA GCC
TCC CAC TAC TTT GAA AGA CAC CTG GAG TTC GAG GCC CGG
ACG CTG TCC CCA GGC CAC ACC TGG GAG GAG GCC CCC CTG
CTG ACT CTC AAG CAG AAG CAG GAA TGG ATC TGC CTG GAG
ACG CTC ACC CCA GAC ACC CAG TAT GAG TTT CAG GTG CGG
GTC AAG CCT CTG CAA GGC GAG TTC ACG ACC TGG AGC CCC
TGG AGC CAG CCC CTG GCC TTC AGG ACA AAG CCT GCA GCC
CTT GGG AAG GAC ACC ATT CCG TGG CTC GGC CAC CTC CTC
GTG GGC CTC AGC GGG GCT TTT GGC TTC ATC ATC TTA GTG
TAC TTG CTG ATC AAC TGC AGG AAC ACC GGG CCA TGG CTG
AAG AAG CTC CTG AAG TGT AAC ACC CCA GAC CCC TCG AAG
TTC TTT TCC CAG CTG AGC TCA GAG CAT GGA GGA GAC GTC
CAG AAG TGG CTC TCT TCG CCC TTC CCC TGA TCG TCC TTC
AGC CCT GGC GGC CTG GCA CCT GAG ATC TCG CCA CTA GAA
GTG CTG GAG AGG GAC AAG GTG ACG CAG CTG CTC CTG CAG
CAG GAC AAG GTG CCT GAG CCC GCA TCC TTA AGC AGC AAC
CAC TCG CTG ACC AGC TGC TTC ACC AAC CAG GGT TAC TTC
TTC TTC CAC CTC CCG GAT GCC TTG GAG ATA GAG GCC TGC
CAG GTG TAC TTT ACT TAC GAC CCC TAC TCA GAG GAA GAC
CCT GAT GAG GGT GTG GCC GGG GCA CCC ACA GGG TCT TCC
CCC CAA CCC CTG CAG CCT CTG TCA GGG GAG GAC GAC GCC
TAC TGC ACC TTC CCC TCC AGG GAT GAC CTG CTG CTC TTC
TCC CCC AGT CTC CTC GGT GGC CCC AGC CCC CCA AGC ACT
GCC CCT GGG GGC AGT GGG GCC GGT GAA GAG AGG ATG CCC
CCT TCT TTG CAA GAA AGA GTC CCC AGA GAC TGG GAC CCC
CAG CCC CTG GGG CCT CCC ACC CCA GGA GTC CCA GAC CTG

GTG GAT TTT CAG CCA CCC CCT GAG CTG GTG CTG CGA GAG
GCT GGG GAG GAG GTC CCT GAC GCT GGC CCC AGG GAG GGA
GTC AGT TTC CCC TGG TCC AGG CCT CCT GGG CAG GGG GAG
TTC AGG GCC CTT AAT GCT CGC CTG CCC CTG AAC ACT GAT
GCC TAC TTG TCC CTC CAA GAA CTC CAG GGT CAG GAC CCA
ATC CAC TTG GTG TAG ACAGATGGCCAGGGTGGGAGGCAGGCAGCT
GCCTGCTCTGCGCCGAGCCTCAGAAGGACCCTGTTGAGGGTCCTCAGTCCA
CTGCTGAGGACACTCAGTGTCCAGTTGCAGCTGGACTTCTCCACCCGGATG
GCCCCCACCCAGTCCTGCACACTTGGTCCATCCATTTCCAAACCTCCACTG
CTGCTCCCGGGTCCTGCTGCCCGAGCCAGGAACTGTGTGTGTTGCAGGGGG
GCAGTAACTCCCCAACTCCCTCGTTAATCACAGGATCCCACGAATTTAGGC
TCAGAAGCATCGCTCCTCTCCAGCCCTGCAGCTATTCACCAATATCAGTCC
TCGCGGCTCTCCAGGGCTCCCTGCCCTGACCTCTTCCCTGGGTTTTCTGCC
CCAGCCTCCTCCTTCCCTCCCCTCCCCGTCCACAGGGCAGCCTGAGCGTGC
TTTCCAAAACCCAAATATGGCCACGCTCCCCCTCGGTTCAAAACCTTGCAC
AGGTCCCACTGCCCTCAGCCCCACTTCTCAGCCTGGTACTTGTACCTCCGG
TGTCGTGTGGGGACATCCCCTTCTGCAATCCTCCCTACCGTCCTCCCGAGC
CACTCAGAGCTCCCTCACACCCCCTCTGTTGCACATGCTATTCCCTGGGGC
TGCTGTGCGCTCCCCCTCATCTAGGTGACAAACTTCCCTGACTCTTCAAGT
GCCGGTTTTGCTTCTCCTGGAGGGAAGCACTGCCTCCCTTAATCTGCCAGA
AACTTCTAGCGTCAGTGCTGGAGGGAGAAGCTGTCAGGGACCCAGGGCGCC
TGGAGAAAGAGGCCCTGTTACTATTCCTTTGGGATCTCTGAGGCCTCAGAG
TGCTTGGCTGCTGTATCTTTAATGCTGGGGCCCAAGTAAGGGCACAGATCC
CCCCGACAAAGTGGATGCCTGCTGCATCTTCCCACAGTGGCTTCACAGACC
CACAAGAGAAGCTGATGGGGAGTAAACCCTGGAGTCCGAGGCCCAGGCAGC
AGCCCCGCCTAGTGGTGGGCCCTGATGCTGCCAGGCCTGGGACCTCCCACT
GCCCCCTCCACTGGAGGGGTCTCCTCTGCAGCTCAGGGACTGGCACACTGG
CCTCCAGAAGGGCAGCTCCACAGGGCAGGGCCTCATTATTTTTCACTGCCC
CAGACACAGTGCCCAACACCCCGTCGTATACCCTGGATGAACGAATTAATT
ACCTGGCACCACCTCGTCTGGGCTCCCTGCGCCTGACATTCACACAGAGAG
GCAGAGTCCCGTGCCCATTAGGTCTGGCATGCCCCCTCCTGCAAGGGGCTC
AACCCCCTACCCCGACCCCTCCACGTATCTTTCCTAGGCAGATCACGTTGC
AATGGCTCAAACAACATTCCACCCCAGCAGGACAGTGACCCCAGTCCCAGC
TAACTCTGACCTGGGAGCCCTCAGGCACCTGCACTTACAGGCCTTGCTCAC

AGCTGATTGGGCACCTGACCACACGCCCCCACAGGCTCTGACCAGCAGCCT
ATGAGGGGGTTTGGCACCAAGCTCTGTCCAATCAGGTAGGCTGGGCCTGAA
CTAGCCAATCAGATCAACTCTGTCTTGGGCGTTTGAACTCAGGGAGGGAGG
CCCTTGGGAGCAGGTGCTTGTGGACAAGGCTCCACAAGCGTTGAGCCTTGG
AAAGGTAGACAAGCGTTGAGCCACTAAGCAGAGGACCTTGGGTTCCCAATA
CAAAAATACCTACTGCTGAGAGGGCTGCTGACCATTTGGTCAGGATTCCTG
TTGCCTTTATATCCAAAATAAACTCCCCTTTCTTGAGGTTGTCTGAGTCTT
GGGTCTATGCCTTGAAAAAAGCTGAATTATTGGACAGTCTCACCTCCTGCC
ATAGGGTCCTGAATGTTTCAGACCACAAGGGGCTCCACACCTTTGCTGTGT
GTTCTGGGGCAACCTACTAATCCTCTCTGCAAGTCGGTCTCCTTATCCCCC
CAAATGGAAATTGTATTTGCCTTCTCCACTTTGGGAGGCTCCCACTTCTTG
GGAGGGTTACATTTTTTAAGTCTTAATCATTTGTGACATATGTATCTATAC
ATCCGTATCTTTTAATGATCCGTGTGTACCATCTTTGTGATTATTTCCTTA
ATATTTTTTCTTTAAGTCAGTTCATTTTCGTTGAAATACATTTATAAAGAA
AAATCTTTGTTACTCTGTAAATGAAAAAACCCATTTTCGCTATAAATAAAA
GGTAACTGTACAAAATAAGTACAAT

or a portion thereof or a degenerate variant thereof.

5. A recombinant DNA molecule according to claim 1 characterized by a structural gene having the formula

ATG

GCT ACC ATA GCT CTT CCC TGG AGC CTG TCC CTC TAC GTC TTC CTC CTG CTC CTG GCT ACA CCT TGG GCA TCT GCA

GCA GTG AAA AAC TGT TCC CAT CTT GAA TGC TTC TAC AAC TCA AGA GCC AAT GTC TCT TGC ATG TGG AGC CAT GAA

GAG GCT CTG AAT GTC ACA ACC TGC CAC GTC CAT GCC AAG TCG AAC CTG CGA CAC TGG AAC AAA ACC TGT GAG CTA

ACT CTT GTG AGG CAG GCA TCC TGG GCC TGC AAC CTG ATC CTC GGG TCG TTC CCA GAG TCC CAG TCA CTG ACC TCC

GTG GAC CTC CTT GAC ATA AAT GTG GTG TGC TGG GAA GAG AAG GGT TGG CGT AGG GTA AAG ACC TGC GAC TTC CAT

CCC TTT GAC AAC CTT CGC CTG GTG GCC CCT CAT TCC CTC CAA GTT CTG CAC ATT GAT ACC CAG AGA TGT AAC ATA

AGC TGG AAG GTC TCC CAG GTC TCT CAC TAC ATT GAA CCA TAC TTG GAA TTT GAG GCC CGT AGA CGT CTT CTG GGC

CAC AGC TGG GAG GAT GCA TCC GTA TTA AGC CTC AAG CAG AGA CAG ACG TGG CTC TTC TTG GAG ATG CTG ATC CCT

AGT ACC TCA TAT GAG GTC CAG GTG AGG GTC AAA GCT CAA CGA AAC AAT ACC GGG ACC TGG AGT CCC TGG AGC CAG

CCC CTG ACC TTT CGG ACA AGG CCA GCA GAT CCC ATG AAG GAG ATC CTC CCC ATG TCA TGG CTC AGA TAC CTT CTG

CTG GTC CTT GGT TGT TTT TCT GGC TTC TTC TCC TGC GTC TAC ATT TTG GTC AAG XXX CGG TAC CTT GGG CCA TGG

CTG AAG ACA GTT CTC AAG TGC CAC ATC CCA GAT CCT TCT GAG TTC TTC TCC CAG CTG AGC TCC CAG CAT GGG GGA

GAC CTT CAG AAA TGG CTC TCC TCG CCT GTC CCC TTG TCC TTC TTC AGC CCC AGT GGC CCT GCC CCT GAG ATC TCT

CCG CTG GAA GTG CTC GAC GGA GAT TCC AAG GCC GTG CAG CTG CTC CTG TTA CAG AAG GAC TCT GCC CCT TTA CCC

TCG CCC AGC GGC CAC TCA CAG GCC AGC TGC TTC ACC AAC CAG GGC TAC TTC TTC TTC CAT CTG CCC AAT GCC TTG

GAG ATC GAA TCC TGC CAG GTG TAC TTC ACC TAT GAC CCC TGT GTG GAA GAG GAG CTG GAG GAG GAT GGG TCA AGG

CTG CCC GAG GGA TCT CCC CAC CCA CCT CTG CTG CCT CTG GCT GGA GAA CAG GAT GAC TAC TGT GCC TTC CCG CCC

AGG GAT GAC CTG CTG CTC TTC TCC CCG AGC CTC AGC ACC CCC AAC ACT GCC TAT GGG GGC AGC AGA GCC CCT GAA

GAA AGA TCT CCA CTC TCC CTG CAT GAG GGA CTT CCC TCC CTA GCA TCC CGT GAC CTG ATG GGC TTA CAG CGC CCT

CTG GAG CGG ATG CCG GAA GGT GAT GGA CAG GGG CTG TCT GCC AAT AGC TCT GGG GAG CAG GCC AGT GTC CCA GAA

GGC AAC CTT CAT GGG CAA GAT CAG GAC AGA GGC CAG GGC CCC ATC CTG ACC CTG AAC ACC GAT GCC TAT CTG TCT

CTT CAA GAA CTA CAG GCC CAA GAT TCA GTC CAC CTA ATA TAG
***

XXX = GGC or TGC

or a degenerate variant thereof or derivative thereof.

6. A recombinant DNA molecule according to claim 1 characterized by an DNA sequence having the formula:

```
                    CGTTTTCTCTCTCTCTTGCTTCTTGCTCTCTTGCTTCTTACACGCTTGCTCCTGAAGATGTAAGAAATAAAGCTTTGCCGCA
GAAGATTCTGGTCTGTGGTGTTCTTCCTGGCCGGTCGTGAGAACGCGTCTAATAACAATTGGTGCCGAAACCCGGACGAGAAAAACTCGGACGAGA
GTTTACATCACCGGTGCAAGGAAGATCCCTCATTCCAGAACCAGAACTGCGGGTCGCGGTAATAAAGGTTCCCGTAAAGCAGACTGTTAAGAAGGATTC
AACTGTATGAATTCAGAACTTTTCAGCTGGGGAACGAGAGATCCAGTGAGTACAGCTTTACGAGGGTTTGCATCCTCAGCTCCTCTCAGCTGTG   ATG

GCT ACC ATA GCT CTT CCC TGG AGC CTG TCC CTC TAC GTC TTC CTC CTG CTC CTG GCT ACA CCT TGG GCA TCT GCA

GCA GTG AAA AAC TGT TCC CAT CTT GAA TGC TTC TAC AAC TCA AGA GCC AAT GTC TCT TGC ATG TGG AGC CAT GAA

GAG GCT CTG AAT GTC ACA ACC TGC CAC GTC CAT GCC AAG TCG AAC CTG CGA CAC TGG AAC AAA ACC TGT GAG CTA

ACT CTT GTG AGG CAG GCA TCC TGG GCC TGC AAC CTG ATC CTC GGG TCG TTC CCA GAG TCC CAG TCA CTG ACC TCC

GTG GAC CTC CTT GAC ATA AAT GTG GTG TGC TGG GAA GAG AAG GGT TGG CGT AGG GTA AAG ACC TGC GAC TTC CAT

CCC TTT GAC AAC CTT CGC CTG GTG GCC CCT CAT TCC CTC CAA GTT CTG CAC ATT GAT ACC CAG AGA TGT AAC ATA

AGC TGG AAG GTC TCC CAG GTC TCT CAC TAC ATT GAA CCA TAC TTG GAA TTT GAG GCC CGT AGA CGT CTT CTG GGC

CAC AGC TGG GAG GAT GCA TCC GTA TTA AGC CTC AAG CAG AGA CAG ACG TGG CTC TTC TTG GAG ATG CTG ATC CCT

AGT ACC TCA TAT GAG GTC CAG GTG AGG GTC AAA GCT CAA CGA AAC AAT ACC GGG ACC TGG AGT CCC TGG AGC CAG

CCC CTG ACC TTT CGG ACA AGG CCA GCA GAT CCC ATG AAG GAG ATC CTC CCC ATG TCA TGG CTC AGA TAC CTT CTG

CTG GTC CTT GGT TGT TTT TCT GGC TTC TTC TCC TGC GTC TAC ATT TTG GTC AAG XXX  CGG TAC CTT GGG CCA TGG
```

```
CTG AAG ACA GTT CTC AAG TGC CAC ATC CCA GAT CCT TCT GAG TTC TTC TCC CAG CTG AGC TCC CAG CAT GGG GGA

GAC CTT CAG AAA TGG CTC TCC TCG CCT GTC CCC TTG TCC TTC TTC AGC CCC AGT GGC CCT GCC CCT GAG ATC TCT

CCG CTG GAA GTG CTC GAC GGA GAT TCC AAG GCC GTG CAG CTG CTC CTG TTA CAG AAG GAC TCT GCC CCT TTA CCC

TCG CCC AGC GGC CAC TCA CAG GCC AGC TGC TTC ACC AAC CAG GGC TAC TTC TTC TTC CAT CTG CCC AAT GCC TTG

GAG ATC GAA TCC TGC CAG GTG TAC TTC ACC TAT GAC CCC TGT GTG GAA GAG GAG GTG GAG GAG GAT GGG TCA AGG

CTG CCC GAG GGA TCT CCC CAC CCA CCT CTG CTG CCT CTG GCT GGA GAA CAG GAT GAC TAC TGT GCC TTC CCG CCC

AGG GAT GAC CTG CTG CTC TTC TCC CCG AGC CTC AGC ACC CCC AAC ACT GCC TAT GGG GGC AGC AGA GCC CCT GAA

GAA AGA TCT CCA CTC TCC CTG CAT GAG GGA CTT CCC TCC CTA GCA TCC CGT GAC CTG ATG GGC TTA CAG CGC CCT

CTG GAG CGG ATG CCG GAA GGT GAT GGA GAG GGG CTG TCT GCC AAT AGC TCT GGG GAG CAG GCC AGT GTC CCA GAA

GGC AAC CTT CAT GGG CAA GAT CAG GAC AGA GGC CAG GGC CCC ATC CTG ACC CTG AAC ACC GAT GCC TAT CTG TCT

CTT CAA GAA CTA CAG GCC CAA GAT TCA GTC CAC CTA ATA TAG   CAGGTGGCCACGACTGGGATCCAGCTGCCTGGATCAGGTCAG
                                                    ***

GCTTGAGGAAGACTGCTTAAGAGGTCTCTTGAGGACAGTCCACTGCTGAGGACTGTCCTGGCTATCCCTGCCCCCCCCCTCCAAACTTAATCATCCACT
TCTGAACTCCATTTGCTACTTCCTGGTCTAACCAGGGTTTGGTGGAGGGTGGGAGTGGGGGAGCGGTGGTCAGCTCCACTGCCCTATTTAGTCATGAG
GTCACTAGCTTCCTGTACCTGCCTCCTTCCCTCCTGCTTGCCTTCACAGGGCAGCTAGAACTTGCTTCCCCG

XXX = GGC or TGC
```

or a degenerate variant thereof.

7. A recombinant DNA molecule as defined in claim 1 coding for a water soluble portion of the human IL-2R$\beta$ chain or a derivative thereof.

8. A recombinant DNA molecule as defined in claim 7 coding for amino acids 1 to 210 of the human IL-2R$\beta$ chain.

9. A recombinant DNA molecule as defined in claim 8 in which the terminal nucleotides are as follows

GCC CTT GCT AGC TAG

and which codes for a derivative of the water soluble human IL-2Rβ chain.

10. A recombinant DNA molecule as defined in any one of claims 1 to 9 which further comprises regulatory sequences operably linked to the structural gene for the IL-2Rβ chain or portion or derivative thereof.

11. A recombinant DNA molecule as defined in claim 10 which is a plasmid.

12. A recombinant DNA molecule as defined in claim 11, this being one of the following

pIL-2Rβ6,

pIL-2Rβ9,

pIL-2Rβ19,

pIL-2Rβ30.

pMIL-2Rβ 36

13. A recombinant DNA molecule capable of hybridising to the cDNA insert of any one of the plasmids listed in claim 12 which codes for IL-2Rβ or a portion thereof, which recombinant DNA molecule codes for a protein having the activity of IL-2Rβ chain or a water soluble portion thereof as defined in claim 8.

14. A host cell which has been transformed by a recombinant DNA molecule as defined in any one of claims 1 to 13.

15. A host cell as defined in claim 14, which is a bacterial cell or a yeast cell or a mammalian cell.

16. A protein having the structure defined by the cDNA set forth in anyone of claims 1 to 9 or 13 or portion thereof.

17. A hybridoma, sub-clone or mutant thereof capable of secreting a monoclonal antibody having a specific affinity to a protein as defined in claim 15.

18. A monoclonal antibody having a specific affinity to a protein as defined in claim 15.

19. A method of producing a hybridoma as defined in claim 16 which comprises immunizing a non-human animal with a protein as defined in claim 15, removing spleen cells from the immunized animal and fusing the spleen cells with non-immunoglobulin secreting myloma cells, and selecting from the resulting hybridomas a cell line which produces a monoclonal antibody having the desired binding specificity and, if desired, subsequently sub-cloning said hybridoma.

Fig. 1 B

```
                                  GCAGCCAGAGCTCAGCAGGGCCCTGGAGAGATGG        34
      CCACGGTCCCAGCACCGGGGAGGACTGGAGAGCGCGCGCTGCCACCGCCCC
      ATGTCTCAGCCAGGGCTTCCTTCCTCGGCTCCACCCTGTGGATGTA  ATG                  134
                                                      Met
                                                      -26
      GCG GCC CCT GCT CTG TCC TGG CGT CTG CCC CTC CTC ATC                  173
-25   Ala Ala Pro Ala Leu Ser Trp Arg Leu Pro Leu Leu Ile

      CTC CTC CTG CCC CTG GCT ACC TCT TGG GCA TCT GCA GCG                  212
-12   Leu Leu Leu Pro Leu Ala Thr Ser Trp Ala Ser Ala Ala

      GTG AAT GGC ACT TCC CAG TTC AGA TGC TTC TAC AAC TCG                  251
2     Val Asn Gly Thr Ser Gln Phe Thr Cys Phe Tyr Asn Ser

      AGA GCC AAC ATC TCC TGT CTC TGG AGC CAA GAT GGG GCT                  290
15    Arg Ala Asn Ile Ser Cys Val Trp Ser Gln Asp Gly Ala

      CTG CAG GAC ACT TCC TGC CAA GTC CAT GCC TGG CCG GAC                  329
28    Leu Gln Asp Thr Ser Cys Gln Val His Ala Trp Pro Asp

      AGA CGG CGG TGG AAC CAA ACC TGT GAG CTG CTC CCC GTG                  368
41    Arg Arg Arg Trp Asn Gln Thr Cys Glu Leu Leu Pro Val

      AGT CAA GCA TCC TGG GCC TGC AAC CTG ATC CTC GGA GCC                  407
54    Ser Gln Ala Ser Trp Ala Cys Asn Leu Ile Leu Gly Ala

      CCA GAT TCT CAG AAA CTG ACC ACA GTT GAC ATC GTC ACC                  446
67    Pro Asp Ser Gln Lys Leu Thr Thr Val Asp Ile Val Thr

      CTG AGG GTG CTG TGC CGT GAG GGG GTG CGA TGG AGG GTG                  485
80    Leu Arg Val Leu Cys Arg Glu Gly Val Arg Trp Arg Val

      ATG GCC ATC CAG GAC TTC AAG CCC TTT GAG AAC CTT CGC                  524
93    Met Ala Ile Gln Asp Phe Lys ProPhe Glu Asn Leu Arg

      CTG ATG GCC CCC ATC TCC CTC CAA GTT GTC CAC GTG GAG                  563
106   Leu Met Ala Pro Ile Ser Leu Gln Val Val His Val Glu

      ACC CAC AGA TGG AAC ATA AGC TGG GAA ATC TCC CAA GCC                  602
119   Thr His Arg Cys Asn Ile Ser Trp Glu Ile Ser Gln Ala

      TCC CAC TAC TTT GAA AGA CAC CTG GAG TTC GAG GCC CGG                  641
132   Ser His Tyr Phe Glu Arg His Leu Glu Phe Glu Ala Arg

      ACG CTG TCC CCA GGC CAC ACC TGG GAG GAG GCC CCC CTG                  680
145   Thr Leu Ser Pro Gly His Thr Trp Glu Glu Ala Pro Leu
```

Fig. 1 B

```
     CTG ACT CTC AAG CAG AAG CAG GAA TGG ATC TGC CTG GAG    719
158  Leu Thr Leu Lys Gln Lys Gln Glu Trp Ile Cys Leu Glu

     ACG CTC ACC CCA GAC ACC CAG TAT GAG TTT CAG GTG CGG    758
171  Thr Leu Thr Pro Asp Thr Gln Tyr Glu Phe Gln Val Arg

     GTC AAG CCT CTG CAA GGC GAG TTC ACG ACC TGG AGC CCC    797
184  Val Lys Pro Leu Gln Gly Glu Phe Thr Thr Trp Ser Pro

     TGG AGC CAG CCC CTG GCC TTC AGG ACA AAG CCT GCA GCC    836
197  Trp Ser Gln Pro Leu Ala Phe Arg Thr Lys Pro Ala Ala

     CTT GGG AAG GAC ACC ATT CCG TGG CTC GGC CAC CTC CTC    875
210  Leu Gly Lys Asp Thr Ile Pro Trp Leu Gly His Leu Leu

     GTG GGC CTC AGC GGG GCT TTT GGC TTC ATC ATC TTA GTG    914
223  Val Gly Leu Ser Gly Ala Phe Gly Phe Ile Ile Leu Val

     TAC TTG CTG ATC AAC TGC AGG AAC ACC GGG CCA TGG CTG    953
236  Tyr Leu Leu Ile Asn Cys Arg Asn Thr Gly Pro Trp Leu

     AAG AAG CTC CTG AAG TGT AAC ACC CCA GAC CCC TCG AAG    992
249  Lys Lys Val Leu Lys Cys Asn Thr Pro Asp Pro Ser Lys

     TTC TTT TCC CAG CTG AGC TCA GAG CAT GGA GGA GAC GTC   1031
262  Phe Phe Ser Gln Leu Ser Ser Glu His Gly Gly Asp Val

     CAG AAG TGG CTC TCT TCG CCC TTC CCC TGA TCG TCC TTC   1070
275  Gln Lys Trp Leu Ser Ser Pro Phe Pro Ser Ser Ser Phe

     AGC CCT GGC GGC CTG GCA CCT GAG ATC TCG CCA CTA GAA   1109
288  Ser Pro Gly Gly Leu Ala Pro Glu Ile Ser Pro Leu Glu

     GTG CTG GAG AGG GAC AAG GTG ACG CAG CTG CTC CTG CAG   1148
301  Val Leu Glu Arg Asp Lys Val Thr Gln Leu Leu Leu Gln

     CAG GAC AAG GTG CCT GAG CCC GCA TCC TTA AGC AGC AAC   1187
314  Gln Asp Lys Val Pro Glu Pro Ala Ser Leu Ser Ser Asn

     CAC TCG CTG ACC AGC TGC TTC ACC AAC CAG GGT TAC TTC   1226
327  His Ser Leu Thr Ser Cys Phe Thr Asn Gln Gly Tyr Phe

     TTC TTC CAC CTC CCG GAT GCC TTG GAG ATA GAG GCC TGC   1265
340  Phe Phe His Leu Pro Asp Ala Leu Glu Ile Glu Ala Cys

     CAG GTG TAC TTT ACT TAC GAC CCC TAC TCA GAG GAA GAC   1304
353  Gln Val Tyr Phe Thr Tyr Asp Pro Tyr Ser Glu Glu Asp

     CCT GAT GAG GGT GTG GCC GGG GCA CCC ACA GGG TCT TCC   1343
366  Pro Asp Glu Gly Val Ala Gly Ala Pro Thr Gly Ser Ser

     CCC CAA CCC CTG CAG CCT CTG TCA GGG GAG GAC GAC GCC   1382
379  Pro Gln Pro Leu Gln Pro Leu Ser Gly Glu Asp Asp Ala
```

Fig. 1 B

```
     TAC TGC ACC TTC CCC TCC AGG GAT GAC CTG CTG CTC TTC   1421
392  Tyr Cys Thr Phe Pro Ser Arg Asp Asp Leu Leu Leu Phe

     TCC CCC AGT CTC CTC GGT GGC CCC AGC CCC CCA AGC ACT   1460
405  Ser Pro Ser Leu Leu Gly Gly Pro Ser Pro Pro Ser Thr

     GCC CCT GGG GGC AGT GGG GCC GGT GAA GAG AGG ATG CCC   1499
418  Ala Pro Gly Gly Ser Gly Ala Gly Glu Glu Arg Met Pro

     CCT TCT TTG CAA GAA AGA GTC CCC AGA GAC TGG GAC CCC   1538
431  Pro Ser Leu Gln Glu Arg Val Pro Arg Asp Trp Asp Pro

     CAG CCC CTG GGG CCT CCC ACC CCA GGA GTC CCA GAC CTG   1577
444  Gln Pro Leu Gly Pro Pro Thr Pro Gly Val Pro Asp Leu

     GTG GAT TTT CAG CCA CCC CCT GAG CTG GTG CTG CGA GAG   1616
457  Val Asp Phe Gln Pro Pro Pro Glu Leu Val Leu Arg Glu

     GCT GGG GAG GAG GTC CCT GAC GCT GGC CCC AGG GAG GGA   1655
470  Ala Gly Glu Glu Val Pro Asp Ala Gly Pro Arg Glu Gly

     GTC AGT TTC CCC TGG TCC AGG CCT CCT GGG CAG GGG GAG   1694
483  Val Ser Phe Pro Trp Ser Arg Pro Pro Gly Gln Gly Glu

     TTC AGG GCC CTT AAT GCT CGC CTG CCC CTG AAC ACT GAT   1733
496  Phe Arg Ala Leu Asn Ala Arg Leu Pro Leu Asn Thr Asp

     GCC TAC TTG TCC CTC CAA GAA CTC CAG GGT CAG GAC CCA   1772
509  Ala Tyr Leu Ser Leu Gln Glu Leu Gln Gly Gln Asp Pro

     ATC CAC TTG GTG TAG  ACAGATGGCCAGGGTGGAGGCAGGCAGCT   1817
522  Thr His Leu Val ***

     GCCTGCTCTGCGCCGAGCCTCAGAAGGACCCTGTTGAGGGTCCTCAGTCCA
     CTGCTGAGGACACTCAGTGTCCAGTTGCAGCTGGACTTCTCCACCCGGATG
     GCCCCCACCCAGTCCTGCACACTTGGTCCATCCATTTCCAAACCTCCACTG
     CTGCTCCCGGGTCCTGCTGCCCGAGCCAGGAACTGTGTGTGTTGCAGGGGG
     GCAGTAACTCCCCAACTCCCTCGTTAATCACAGGATCCCACGAATTTAGGC
     TCAGAAGCATCGCTCCTCTCCAGCCCTGCAGCTATTCACCAATATCAGTCC
     TCGCGGCTCTCCAGGGCTCCCTGCCCTGACCTCTTCCCTGGGTTTTCTGCC
     CCAGCCTCCTCCTTCCCTCCCCTCCCCGTCCACAGGGCAGCCTGAGCGTGC
     TTTCCAAAACCCAAATATGGCCACGCTCCCCCTCGGTTCAAAACCTTGCAC
     AGGTCCCACTGCCCTCAGCCCCACTTCTCAGCCTGGTACTTGTACCTCCGG
     TGTCGTGTGGGGACATCCCCTTCTGCAATCCTCCCTACCGTCCTCCCGAGC
     CACTCAGAGCTCCCTCACACCCCCTCTGTTGCACATGCTATTCCCTGGGGC
     TGCTGTGCGCTCCCCCTCATCTAGGTGACAAACTTCCCTGACTCTTCAAGT
```

Fig. 1 B

GCCGGTTTTGCTTCTCCTGGAGGGAAGCACTGCCTCCCTTAATCTGCCAGA
AACTTCTAGCGTCAGTGCTGGAGGGAGAAGCTGTCAGGGACCCAGGGCGCC
TGGAGAAAGAGGCCCTGTTACTATTCCTTTGGGATCTCTGAGGCCTCAGAG
TGCTTGGCTGCTGTATCTTTAATGCTGGGGCCCAAGTAAGGGCACAGATCC
CCCCGACAAAGTGGATGCCTGCTGCATCTTCCCACAGTGGCTTCACAGACC
CACAAGAGAAGCTGATGGGGAGTAAACCCTGGAGTCCGAGGCCCAGGCAGC
AGCCCCGCCTAGTGGTGGGCCCTGATGCTGCCAGGCCTGGGACCTCCCACT
GCCCCCTCCACTGGAGGGGTCTCCTCTGCAGCTCAGGGACTGGCACACTGG
CCTCCAGAAGGGCAGCTCCACAGGGCAGGGCCTCATTATTTTTCACTGCCC
CAGACACAGTGCCCAACACCCCGTCGTATACCCTGGATGAACGAATTAATT
ACCTGGCACCACCTCGTCTGGGCTCCCTGCGCCTGACATTCACACAGAGAG
GCAGAGTCCCGTGCCCATTAGGTCTGGCATGCCCCCTCCTGCAAGGGGCTC
AACCCCCTACCCCGACCCCTCCACGTATCTTTCCTAGGCAGATCACGTTGC
AATGGCTCAAACAACATTCCACCCCAGCAGGACAGTGACCCCAGTCCCAGC
TAACTCTGACCTGGGAGCCCTCAGGCACCTGCACTTACAGGCCTTGCTCAC
AGCTGATTGGGCACCTGACCACACGCCCCCACAGGCTCTGACCAGCAGCCT
ATGAGGGGGTTTGGCACCAAGCTCTGTCCAATCAGGTAGGCTGGGCCTGAA
CTAGCCAATCAGATCAACTCTGTCTTGGGCGTTTGAACTCAGGGAGGGAGG
CCCTTGGGAGCAGGTGCTTGTGGACAAGGCTCCACAAGCGTTGAGCCTTGG
AAAGGTAGACAAGCGTTGAGCCACTAAGCAGAGGACCTTGGGTTCCCAATA
CAAAAATACCTACTGCTGAGAGGGCTGCTGACCATTTGGTCAGGATTCCTG
TTGCCTTTATATCCAAAATAAACTCCCCTTTCTTGAGGTTGTCTGAGTCTT
GGGTCTATGCCTTGAAAAAAGCTGAATTATTGGACAGTCTCACCTCCTGCC
ATAGGGTCCTGAATGTTTCAGACCACAAGGGGCTCCACACCTTTGCTGTGT
GTTCTGGGGCAACCTACTAATCCTCTCTGCAAGTCGGTCTCCTTATCCCCC
CAAATGGAAATTGTATTTGCCTTCTCCACTTTGGGAGGCTCCCACTTCTTG
GGAGGGTTACATTTTTTAAGTCTTAATCATTTGTGACATATGTATCTATAC
ATCCGTATCTTTTAATGATCCGTGTGTACCATCTTTGTGATTATTTCCTTA
ATATTTTTTCTTTAAGTCAGTTCATTTTCGTTGAAATACATTTATAAAGAA
AAATCTTTGTTACTCTGTAAATGAAAAAACCCATTTTCGCTATAAATAAAA
GGTAACTGTACAAAATAAGTACAAT 4034

FIG. 2
(cell length:19)
IL-2Rα
IL-2Rβ
SG
TM
SG
TM
Hydropathy index
+ 2.5
0
- 2.5
+ 2.5
0
-2.5
Amino acids
0
100
200
300
400
500

FIG. 3a

1 2 3 4 5 6 7 8 9
28 s
18 s

FIG. 3b

1 2 3 4 5 6
28 s
18 s

FIG.4a

Relative cell number
(1) ELβ-13
(2) Jβ-8
(3) Jα-5
(4) Jαβ -2
(5) Jαβ-10
(6) Fβ-3
Relative fluorescence intensity (log scale

Bound IL-2/free IL-2
(1) EL$\beta$-13
(2) J$\beta$-8
(3) J$\alpha$-5
(4) J$\alpha\beta$-2
(5) J$\alpha\beta$-10
Bound IL-2 molecules per cell (×10⁻³)

FIG.4b

125I-IL-2 :
anti-Tac
Mik-β1
cold IL-2
5 nM
100pM
kd
200
92
66
45
β+IL-2
α+IL-2
M 1 2 3 4 5 6 7 8 9 10
M 11 12 13 14 15 16

FIG. 5

FIG. 6

(a)
ELβ-13
(b)
Jβ-8
(c)
Jαβ-10
(d)
Jα-5
1.5
1
0.5
3
2
1
2
1
2
1
$^{125}$I-IL-2 (cpm x 10$^{-3}$)
0
15
30
45
60
0
15
30
45
60
Incubation time (min)

Fig. 7

Restriction enzyme leavage map of the mouse IL-2 receptor ß chain cDNA clone

Hind III
Xba I
Xho I
Sma I
Eco RI
Pst I
Aat II
Nde I
Kpn I
TM
Sac I
Bgl II
Bgl II
Hha I
Bam HI
Xho I
Pst I
Not I
Xba I
SP
EC
CP
ORF (1617bp)
cDNA (2306bp)

Fig. 8

```
                CGTTTTCTCTCTCTCTTGCTTCTTGCTCTCTTGCTTCTTACACGCTTGCTCCTGAAGATGTAAGAAATAAAGCTTTGCCGCA   82
     GAAGATTCTGGTCTGTCGTGTTCTTCCTGGCCGGTCGTGAGAACGCGTCTAATAACAATTGGTGCCGAAACCCGGGACGAGAAAAAACTCGGCACGAGA  181
     GTTTACATCACCGGTGCAAGGAAGATCCCTCATTCCAGAACCAGAACTGCGGGTCGCGGTAATAAAGGTTCCCGTAAAGCAGACTGTTAAGAAGGATTC  280
     AACTGTATGAATTCAGAACTTTTCAGCTGGGGAACGAGAGATCCAGTGAGTACAGCTTTACGAGGGTTTGCATCCTCAGCTCCTCTCAGCTGTG   ATG  377
                                                                                                      Met

     GCT ACC ATA GCT CTT CCC TGG AGC CTG TCC CTC TAC GTC TTC CTC CTG CTC CTG GCT ACA CCT TGG GCA TCT GCA  452
     Ala Thr Ile Ala Leu Pro Trp Ser Leu Ser Leu Tyr Val Phe Leu Leu Leu Leu Ala Thr Pro Trp Ala Ser Ala

1    GCA GTG AAA AAC TGT TCC CAT CTT GAA TGC TTC TAC AAC TCA AGA GCC AAT GTC TCT TGC ATG TGG AGC CAT GAA  527
     Ala Val Lys Asn Cys Ser His Leu Glu Cys Phe Tyr Asn Ser Arg Ala Asn Val Ser Cys Met Trp Ser His Glu

26   GAG GCT CTG AAT GTC ACA ACC TGC CAC GTC CAT GCC AAG TCG AAC CTG CGA CAC TGG AAC AAA ACC TGT GAG CTA  602
     Glu Ala Leu Asn Val Thr Thr Cys His Val His Ala Lys Ser Asn Leu Arg His Trp Asn Lys Thr Cys Glu Leu

51   ACT CTT GTG AGG CAG GCA TCC TGG GCC TGC AAC CTG ATC CTC GGG TCG TTC CCA GAG TCC CAG TCA CTG ACC TCC  677
     Thr Leu Val Arg Gln Ala Ser Trp Ala Cys Asn Leu Ile Leu Gly Ser Phe Pro Glu Ser Gln Ser Leu Thr Ser

76   GTG GAC CTC CTT GAC ATA AAT GTG GTG TGC TGG GAA GAG AAG GGT TGG CGT AGG GTA AAG ACC TGC GAC TTC CAT  752
     Val Asp Leu Leu Asp Ile Asn Val Val Cys Trp Glu Glu Lys Gly Trp Arg Arg Val Lys Thr Cys Asp Phe His

101  CCC TTT GAC AAC CTT CGC CTG GTG GCC CCT CAT TCC CTC CAA GTT CTG CAC ATT GAT ACC CAG AGA TGT AAC ATA  827
     Pro Phe Asp Asn Leu Arg Leu Val Ala Pro His Ser Leu Gln Val Leu His Ile Asp Thr Gln Arg Cys Asn Ile

126  AGC TGG AAG GTC TCC CAG GTC TCT CAC TAC ATT GAA CCA TAC TTG GAA TTT GAG GCC CGT AGA CGT CTT CTG GGC  902
     Ser Trp Lys Val Ser Gln Val Ser His Tyr Ile Glu Pro Tyr Leu Glu Phe Glu Ala Arg Arg Arg Leu Leu Gly

151  CAC AGC TGG GAG GAT GCA TCC GTA TTA AGC CTC AAG CAG AGA CAG ACG TGG CTC TTC TTG GAG ATG CTG ATC CCT  977
     His Ser Trp Glu Asp Ala Ser Val Leu Ser Leu Lys Gln Arg Gln Gln Trp Leu Phe Leu Glu Met Leu Ile Pro

176  AGT ACC TCA TAT GAG GTC CAG GTG AGG GTC AAA GCT CAA CGA AAC AAT ACC GGG ACC TGG AGT CCC TGG AGC CAG  1052
     Ser Thr Ser Tyr Glu Val Gln Val Arg Val Lys Ala Gln Arg Asn Asn Thr Gly Thr Trp Ser Pro Trp Ser Gln

201  CCC CTG ACC TTT CGG ACA AGG CCA GCA GAT CCC ATG AAG GAG ATC CTC CCC ATG TCA TGG CTC AGA TAC CTT CTG  1127
     Pro Leu Thr Phe Arg Thr Arg Pro Ala Asp Pro Met Lys Glu Ile Leu Pro Met Ser Trp Leu Arg Tyr Leu Leu

226  CTG GTC CTT GGT TGT TTT TCT GGC TTC TTC TCC TGC GTC TAC ATT TTG GTC AAG XXX CGG TAC CTT GGG CCA TGG  1202
     Leu Val Leu Gly Cys Phe Ser Gly Phe Phe Ser Cys Val Tyr Ile Leu Val Lys Y   Arg Tyr Leu Gly Pro Trp
```

con. Fig. 8

```
251  CTG AAG ACA GTT CTC AAG TGC CAC ATC CCA GAT CCT TCT GAG TTC TTC TCC CAG CTG AGC TCC CAG CAT GGG GGA  1277
     Leu Lys Thr Val Leu Lys Cys His Ile Pro Asp Pro Ser Glu Phe Phe Ser Gln Leu Ser Ser Gln His Gly Gly

276  GAC CTT CAG AAA TGG CTC TCC TCG CCT GTC CCC TTG TCC TTC TTC AGC CCC AGT GGC CCT GCC CCT GAG ATC TCT  1352
     Asp Leu Gln Lys Trp Leu Ser Ser Pro Val Pro Leu Ser Phe Phe Ser Pro Ser Gly Pro Ala Pro Glu Ile Ser

301  CCG CTG GAA GTG CTC GAC GGA GAT TCC AAG GCC GTG CAG CTG CTC CTG TTA CAG AAG GAC TCT GCC CCT TTA CCC  1427
     Pro Leu Glu Val Leu Asp Gly Asp Ser Lys Ala Val Gln Leu Leu Leu Leu Gln Lys Asp Ser Ala Pro Leu Pro

326  TCG CCC AGC GGC CAC TCA CAG GCC AGC TGC TTC ACC AAC CAG GGC TAC TTC TTC TTC CAT CTG CCC AAT GCC TTG  1502
     Ser Pro Ser Gly His Ser Gln Ala Ser Cys Phe Thr Asn Gln Gly Tyr Phe Phe Phe His Leu Pro Asn Ala Leu

351  GAG ATC GAA TCC TGC CAG GTG TAC TTC ACC TAT GAC CCC TGT GTG GAA GAG GAG GTG GAG GAG GAT GGG TCA AGG  1577
     Glu Ile Glu Ser Cys Gln Val Tyr Phe Thr Tyr Asp Pro Cys Val Glu Glu Glu Val Glu Glu Asp Gly Ser Arg

376  CTG CCC GAG GGA TCT CCC CAC CCA CCT CTG CTG CCT CTG GCT GGA GAA CAG GAT GAC TAC TGT GCC TTC CCG CCC  1652
     Leu Pro Glu Gly Ser Pro His Pro Pro Leu Leu Pro Leu Ala Gly Glu Gln Asp Asp Tyr Cys Ala Phe Pro Pro

401  AGG GAT GAC CTG CTG CTC TTC TCC CCG AGC CTC AGC ACC CCC AAC ACT GCC TAT GGG GGC AGC AGA GCC CCT GAA  1727
     Arg Asp Asp Leu Leu Leu Phe Ser Pro Ser Leu Ser Thr Pro Asn Thr Ala Tyr Gly Gly Ser Arg Ala Pro Glu

426  GAA AGA TCT CCA CTC TCC CTG CAT GAG GGA CTT CCC TCC CTA GCA TCC CGT GAC CTG ATG GGC TTA CAG CGC CCT  1802
     Glu Arg Ser Pro Leu Ser Leu His Glu Gly Leu Pro Ser Leu Ala Ser Arg Asp Leu Met Gly Leu Gln Arg Pro

451  CTG GAG CGG ATG CCG GAA GGT GAT GGA GAG GGG CTG TCT GCC AAT AGC TCT GGG GAG CAG GCC AGT GTC CCA GAA  1877
     Leu Glu Arg Met Pro Glu Gly Asp Gly Glu Gly Leu Ser Ala Asn Ser Ser Gly Glu Gln Ala Ser Val Pro Glu

476  GGC AAC CTT CAT GGG CAA GAT CAG GAC AGA GGC CAG GGC CCC ATC CTG ACC CTG AAC ACC GAT GCC TAT CTG TCT  1952
     Gly Asn Leu His Gly Gln Asp Gln Asp Arg Gly Gln Gly Pro Ile Leu Thr Leu Asn Thr Asp Ala Tyr Leu Ser

501  CTT CAA GAA CTA CAG GCC CAA GAT TCA GTC CAC CTA ATA TAG  CAGGTGGCCAGGACTGGGATCCAGCTGCCTGGATCAGGTCAG  2036
     Leu Gln Glu Leu Gln Ala Gln Asp Ser Val His Leu Ile ***

     GCTTGAGGAAGACTGCTTAAGAGGTCTCTTGAGGACAGTCCACTGCTGAGGACTGTCCTGGCTATCCCTGCCCCCCCCCTCCAAACTTAATCATCCACT  2135
     TCTGAACTCCATTTGCTACTTCCTGGTCTAACCAGGGTTTGGTGGAGGGTGGGGAGTGGGGGAGCGGTGGTCAGCTCCACTGCCCTATTTAGTCATGAG  2234
     GTCACTAGCTTCCTGTACCTGCCTCCTTCCCTCCTGCTTGCCTTCACAGGGCAGCTAGAACTTGCTTCCCCG                             2306
```

XXX = GGC or TGC

Y = Gly or Cys

Figure 9, Page 1

```
        10        20        30        40        50        60
CTCAGCAGGGCCTGGAGAGATGGCCACGGTCCCAGCACCGGGGAGGACTGGAGAGCGCGC

        70        80        90       100       110       120
GCTGCCACCGCCCCATGTCTCAGCCAGGGCTTCCTTCCTCGGCTCCACCCTGTGGATGTA

       130       140       150       160       170       180
ATGGCGGCCCCTGCTCTGTCCTGGCGTCTGCCCCTCCTCATCCTCCTCCTGCCCCTGGCT
MetAlaAlaProAlaLeuSerTrpArgLeuProLeuLeuIleLeuLeuLeuProLeuAla
                        signal peptide
       190       200       210       220       230       240
ACCTCTTGGGCATCTGCAGCGGTGAATGGCACTTCCCAGTTCACATGCTTCTACAACTCG
ThrSerTrpAlaSerAlaAlaValAsnGlyThrSerGlnPheThrCysPheTyrAsnSer

       250       260       270       280       290       300
AGAGCCAACATCTCCTGTGTCTGGAGCCAAGATGGGGCTCTGCAGGACACTTCCTGCCAA
ArgAlaAsnIleSerCysValTrpSerGlnAspGlyAlaLeuGlnAspThrSerCysGln

       310       320       330       340       350       360
GTCCATGCCTGGCCGGACAGACGGCGGTGGAACCAAACCTGTGAGCTGCTCCCCGTGAGT
ValHisAlaTrpProAspArgArgArgTrpAsnGlnThrCysGluLeuLeuProValSer

       370       380       390       400       410       420
CAAGCATCCTGGGCCTGCAACCTGATCCTCGGAGCCCCAGATTCTCAGAAACTGACCACA
GlnAlaSerTrpAlaCysAsnLeuIleLeuGlyAlaProAspSerGlnLysLeuThrThr

       430       440       450       460       470       480
GTTGACATCGTCACCCTGAGGGTGCTGTGCCGTGAGGGGGTGCGATGGAGGGTGATGGCC
ValAspIleValThrLeuArgValLeuCysArgGluGlyValArgTrpArgValMetAla

       490       500       510       520       530       540
ATCCAGGACTTCAAGCCCTTTGAGAACCTTCGCCTGATGGCCCCCATCTCCCTCCAAGTT
IleGlnAspPheLysProPheGluAsnLeuArgLeuMetAlaProIleSerLeuGlnVal

       550       560       570       580       590       600
GTCCACGTGGAGACCCACAGATGCAACATAAGCTGGGAAATCTCCCAAGCCTCCCACTAC
ValHisValGluThrHisArgCysAsnIleSerTrpGluIleSerGlnAlaSerHisTyr
```

Figure 9, Page 2

```
     610        620        630        640        650        660
TTTGAAAGACACCTGGAGTTCGAGGCCCGGACGCTGTCCCCAGGCCACACCTGGGAGGAG
PheGluArgHisLeuGluPheGluAlaArgThrLeuSerProGlyHisThrTrpGluGlu

     670        680        690        700        710        720
GCCCCCCTGCTGACTCTCAAGCAGAAGCAGGAATGGATCTGCCTGGAGACGCTCACCCCA
AlaProLeuLeuThrLeuLysGlnLysGlnGluTrpIleCysLeuGluThrLeuThrPro

     730        740        750        760        770        780
GACACCCAGTATGAGTTTCAGGTGCGGGTCAAGCCTCTGCAAGGCGAGTTCACGACCTGG
AspThrGlnTyrGluPheGlnValArgValLysProLeuGlnGlyGluPheThrThrTrp

                                               StyI
     790        800        810        820        830        840
AGCCCCTGGAGCCAGCCCCTGGCCTTCAGGACAAAGCCTGCAGCCCTTGGGAAGGACACC
SerProTrpSerGlnProLeuAlaPheArgThrLysProAlaAlaLeuGlyLysAspThr

     850        860        870        880        890        900
ATTCCGTGGCTCGGCCACCTCCTCGTGGGCCTCAGCGGGGCTTTTGGCTTCATCATCTTA
IleProTrpLeuGlyHisLeuLeuValGlyLeuSerGlyAlaPheGlyPheIleIleLeu
                       Transmembrane

     910        920        930        940        950        960
GTGTACTTGCTGATCAACTGCAGGAACACCGGGCCATGGCTGAAGAAGGTCCTGAAGTGT
ValTyrLeuLeuIleAsnCysArgAsnThrGlyProTrpLeuLysLysValLeuLysCys

     970        980        990       1000       1010       1020
AACACCCCAGACCCCTCGAAGTTCTTTTCCCAGCTGAGCTCAGAGCATGGAGGAGACGTC
AsnThrProAspProSerLysPhePheSerGlnLeuSerSerGluHisGlyGlyAspVal

    1030       1040       1050       1060       1070       1080
CAGAAGTGGCTCTCTTCGCCCTTCCCCTCATCGTCCTTCAGCCCTGGCGGCCTGGCACCT
GlnLysTrpLeuSerSerProPheProSerSerSerPheSerProGlyGlyLeuAlaPro

    1090       1100       1110       1120       1130       1140
GAGATCTCGCCACTAGAAGTGCTGGAGAGGGACAAGGTGACGCAGCTGCTCCTGCAGCAG
GluIleSerProLeuGluValLeuGluArgAspLysValThrGlnLeuLeuLeuGlnGln

    1150       1160       1170       1180       1190       1200
GACAAGGTGCCTGAGCCCGCATCCTTAAGCAGCAACCACTCGCTGACCAGCTGCTTCACC
AspLysValProGluProAlaSerLeuSerSerAsnHisSerLeuThrSerCysPheThr

    1210       1220       1230       1240       1250       1260
AACCAGGGTTACTTCTTCTTCCACCTCCCGGATGCCTTGGAGATAGAGGCCTGCCAGGTG
AsnGlnGlyTyrPhePhePheHisLeuProAspAlaLeuGluIleGluAlaCysGlnVal

    1270       1280       1290       1300       1310       1320
TACTTTACTTACGACCCCTACTCAGAGGAAGACCCTGATGAGGGTGTGGCCGGGGCACCC
TyrPheThrTyrAspProTyrSerGluGluAspProAspGluGlyValAlaGlyAlaPro
```

Figure 9, Page 3

```
       1330      1340      1350      1360      1370      1380
ACAGGGTCTTCCCCCCAACCCCTGCAGCCTCTGTCAGGGGAGGACGACGCCTACTGCACC
ThrGlySerSerProGlnProLeuGlnProLeuSerGlyGluAspAspAlaTyrCysThr

       1390      1400      1410      1420      1430      1440
TTCCCCTCCAGGGATGACCTGCTGCTCTTCTCCCCCAGTCTCCTCGGTGGCCCCAGCCCC
PheProSerArgAspAspLeuLeuLeuPheSerProSerLeuLeuGlyGlyProSerPro

       1450      1460      1470      1480      1490      1500
CCAAGCACTGCCCCTGGGGGCAGTGGGGCCGGTGAAGAGAGGATGCCCCCTTCTTTGCAA
ProSerThrAlaProGlyGlySerGlyAlaGlyGluGluArgMetProProSerLeuGln

       1510      1520      1530      1540      1550      1560
GAAAGAGTCCCCAGAGACTGGGACCCCCAGCCCCTGGGGCCTCCCACCCCAGGAGTCCCA
GluArgValProArgAspTrpAspProGlnProLeuGlyProProThrProGlyValPro

       1570      1580      1590      1600      1610      1620
GACCTGGTGGATTTTCAGCCACCCCCTGAGCTGGTGCTGCGAGAGGCTGGGGAGGAGGTC
AspLeuValAspPheGlnProProProGluLeuValLeuArgGluAlaGlyGluGluVal

       1630      1640      1650      1660      1670      1680
CCTGACGCTGGCCCCAGGGAGGGAGTCAGTTTCCCCTGGTCCAGGCCTCCTGGGCAGGGG
ProAspAlaGlyProArgGluGlyValSerPheProTrpSerArgProProGlyGlnGly

       1690      1700      1710      1720      1730      1740
GAGTTCAGGGCCCTTAATGCTCGCCTGCCCCTGAACACTGATGCCTACTTGTCCCTCCAA
GluPheArgAlaLeuAsnAlaArgLeuProLeuAsnThrAspAlaTyrLeuSerLeuGln

       1750      1760      1770      1780      1790      1800
GAACTCCAGGGTCAGGACCCAACTCACTTGGTGTAGACAGATGGCCAGGGTGGGAGGCAG
GluLeuGlnGlyGlnAspProThrHisLeuVal***

       1810      1820      1830      1840      1850      1860
GCAGCTGCCTGCTCTGCGCCGAGCCTCAGAAGGACCCTGTTGAGGGTCCTCAGTCCACTG

       1870      1880      1890      1900      1910      1920
CTGAGGACACTCAGTGTCCAGTTGCAGCTGGACTTCTCCACCCGGATGGCCCCCACCCAG

       1930      1940      1950      1960      1970      1980
TCCTGCACACTTGGTCCATCCATTTCCAAACCTCCACTGCTGCTCCCGGGTCCTGCTGCC

       1990      2000      2010      2020      2030      2040
CGAGCCAGGAACTGTGTGTGTTGCAGGGGGGCAGTAACTCCCCAACTCCCTCGTTAATCA

       2050      2060      2070      2080      2090      2100
CAGGATCCCACGAATTTAGGCTCAGAAGCATCGCTCCTCTCCAGCCCTGCAGCTATTCAC

       2110      2120      2130      2140      2150      2160
CAATATCAGTCCTCGCGGCTCTCCAGGGCTCCCTGCCCTGACCTCTTCCCTGGGTTTTCT
```

Fig 10
XbrI
BssHII
SmaI
XbaI
β1.9
CDM8
↓BssHII/SmaI
β1.9
fill in
SmaI
AmpR
pBluescript SK
SmaI
CIP
ligation
(BssHII/SmaI)
StyI
StyI
StyI
SmaI
pBluescript SK β1.9
StyI/SmaI
0.8Kb
StyI
SmaI
Sol.β
fill-in
NheI termination linker
NheI
(StyI)
(SmaI)
Sol.β
SalI
NotI
ligation
SalI/NotI
Sol.β
XhoI
NotI
CMVp
Int
(A)
Neo
AmpR
BCMGNeo
BPV
XhoI/NotI
(XhoI/SalI)
NotI
CMVp
Int
Sol.β
(A)
BCMGNeo-Sol β

Fig. 11
Native β
extracellular
GCC CTT GGG AAG GAC ACC ATT CCG
—Ala — Leu ... Gly — Lys — Asp — Thr — Ile — Pro —
240
memb.
Soluble β
GCC CTT GCT AGC TAG
—Ala—Leu—Ala—Ser END
signal peptide
extracellular
memb.
intra cytoplasmic
26
214 aa
25
286
26
212

Al–P
Avidin
Mik–β1
β
Mik–β3

Fig. 12

Fig. 13
soluble β
35S-meth. labeling
Mr x 10-3
69
46
30
sample 2 µl
4 µl
8 µl
← 37kD
1 2 3 4 5 6
mik-β1
upc10
mik-β1
upc10
mik-β1
upc10

Fig 14
Absorbance at 405 nm
1.2
0.8
0.4
$10^{-11}$
$10^{-9}$
$10^{-7}$
$10^{-5}$
Concentration of competitors (M)
unlabeled
Mik-β1
IL-2
IL-2
Mik-β1
Al-p
β
Mik-β3

European Patent Office

# EUROPEAN SEARCH REPORT

Application Number

EP 89 11 3310

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.5) |
|---|---|---|---|
| X | SCIENCE vol. 244, 5 May 1989, pages 551-556, Washington, DC, US; M. HATAKEYAMA et al.: "Interleukin-2 Receptor beta Chain Gene: Generation of Three Receptor Forms by Cloned Human alpha and beta Chain cDNA's" * whole article * | 1-4,7-10 | C 12 N 15/12<br>C 12 P 21/02 |
| Y | idem | 5,6 | |
| X | IMMUNOBIOLOGY vol. 175, no. 4, 1987, pages 343,344, Stuttgart, DE; G. KRUPPA et al.: "Transfer and expression of the human IL 2 receptor beta-chain gene in murine fibroblasts and T lymphoid cells using retroviral vectors" * abstract * | 1,2,10 | |
| Y | CHEMICAL ABSTRACTS vol. 108, no. 3, 18 January 1988, page 462, column 1, abstract no. 20332m, Columbus, Ohio, USA; T. HERRMANN et al.: "The mouse high affinity IL 2 receptor complex. I. Evidence for a third molecule, the putative gamma-chain, associated with the alpha- and/or beta-chain of the receptor" & Immunobiology 1987, vol. 175, no. 3, pages 145-158 * abstract * | 5,6 | TECHNICAL FIELDS SEARCHED (Int. Cl.5)<br>C 12 N 15/12<br>C 12 P 21/02<br>C 12 P 21/00<br>C 12 N 15/24<br>C 12 N 15/26 |
| Y | WO-A-8 900 168 (THE UNITED STATES OF AMERICA) * whole document, in particular page 8, lines 5-30; claims 1,3 * | 1,2,10 | |
| Y | EP-A-0 162 699 (IMMUNEX CORP.) * whole document * -/- | 1,2,10 | |

The present search report has been drawn up for all claims

| Place of search | Date of completion of the search | Examiner |
|---|---|---|
| BERLIN | 02-03-1990 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 89 11 3310

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF EXPERIMENTAL MEDICINE vol. 167, no. 3, March 1988, pages 1265-1270, New York, US; M. SHARON et al.: "The Human Interleukin 2 Receptor beta-Chain (p70). Direct Identification, Partial Purification, and Patterns of Expression on Peripheral Blood Mononuclear Cells" * abstract; pages 1266,1269 * --- | 1,2 | |
| A | CHEMICAL ABSTRACTS vol. 110, no. 17, 24 April 1989, page 563, column 1, abstract no. 152506x, Columbus, Ohio, US; J.C. GUTIERREZ-RAMOS et al.: "Expression of the p75 interleukin 2-binding protein on CD3+4-8-Tac-cells from autoimmune MRL/MP-lpr/lpr mice" & Eur. J. Immunol. 1989, vol. 19, no. 1, pages 201-204 * abstract * --- | 5,6 | |
| | | | TECHNICAL FIELDS SEARCHED (Int. Cl.5) |
| D,A | PROC. NATL. ACAD. SCI. USA vol. 86, March 1989, pages 1982-1986, Washington, DC, US; M. TSUDO et al.: "Characterization of the interleukin 2 receptor beta chain using three distinct monoclonal antibodies" * whole article * --- | 1,2 | |
| A | EP-A-0 202 975 (THE UNITED STATES OF AMERICA) * whole document,in particular column 1, lines 13-53 * ----- | 7 | |

The present search report has been drawn up for all claims

| Place of search | Date of completion of the search | Examiner |
|---|---|---|
| BERLIN | 02-03-1990 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document